(19) Europäisches Patentamt
European Patent Office
Office européen des brevets

(11) EP 3 399 912 B1

(12) **EUROPEAN PATENT SPECIFICATION**

(45) Date of publication and mention
of the grant of the patent:
**13.04.2022 Bulletin 2022/15**

(21) Application number: **17735774.6**

(22) Date of filing: **09.01.2017**

(51) International Patent Classification (IPC):
**A61B 5/11** (2006.01)   **G01P 13/00** (2006.01)

(52) Cooperative Patent Classification (CPC):
**A61B 5/4815; A61B 5/1118; A61B 5/4809;**
**A61B 5/681; A61B 5/7264;** A61B 5/4082;
A61B 5/4812; A61B 2505/07; A61B 2562/0219;
G16H 50/20

(86) International application number:
**PCT/AU2017/050015**

(87) International publication number:
**WO 2017/117636 (13.07.2017 Gazette 2017/28)**

(54) **SYSTEM AND METHOD FOR ASSESSING SLEEP STATE**

SYSTEM UND VERFAHREN ZUR BEURTEILUNG EINES SCHLAFZUSTANDS

SYSTÈME ET PROCÉDÉ D'ÉVALUATION DE L'ÉTAT DU SOMMEIL

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **07.01.2016 AU 2016900036**

(43) Date of publication of application:
**14.11.2018 Bulletin 2018/46**

(73) Proprietor: **Global Kinetics Pty Ltd**
**Melbourne, Victoria 3000 (AU)**

(72) Inventor: **HORNE, Malcolm Kenneth**
**Melbourne**
**Victoria 3000 (AU)**

(74) Representative: **Brevalex**
**95, rue d'Amsterdam**
**75378 Paris Cedex 8 (FR)**

(56) References cited:
**WO-A1-01/95801        WO-A1-2015/118534**
**US-A1- 2004 087 878   US-A1- 2006 020 178**
**US-A1- 2014 364 770**

- **JENNIFER GIRSCHIK ET AL: "Validation of Self-Reported Sleep Against Actigraphy",** JOURNAL OF EPIDEMIOLOGY, vol. 22, no. 5, 28 July 2012 (2012-07-28), pages 462-468, XP055583208, JP ISSN: 0917-5040, DOI: 10.2188/jea.JE20120012
- **STAVITSKY K ET AL: "Sleep in Parkinson's disease: A comparison of actigraphy and subjective measures", PARKINSONISM AND RELATED DISORDERS, ELSEVIER SCIENCE, OXFORD, GB, vol. 16, no. 4, 1 May 2010 (2010-05-01), pages 280-283, XP027017596, ISSN: 1353-8020 [retrieved on 2010-03-03]**
- **MAGLIONE et al.: "Actigraphy for the Assessment of Sleep Measures in Parkinson's Disease", Sleep, vol. 36, no. 8, 1 August 2013 (2013-08-01) , pages 1209-1217, XP055396526,**
- **PAN et al.: Quantitative Evaluation of the Use of Actigraphy for Neurological and Psychiatric Disorders Behavioural Neurology, vol. 2014, 19 August 2014 (2014-08-19), XP055396530,**
- **BHIDAYASIRI et al.: "Capturing Nighttime Symptoms in Parkinson Disease: Technical Development and Experimental Verification of Inertial Sensors for Nocturnal Hypokinesia", JRRD, vol. 53, no. 4, 1 January 2016 (2016-01-01), pages 487-498, XP055570813, DOI: 10.1682/JRRD.2015.04.0062**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Cross-Reference to Related Applications

This application claims the benefit of Australian Provisional Patent Application No. 2016900036 filed 7 January 2016.

Technical Field

The present invention relates to a system and method for monitoring or assessing a sleep state of an individual, and in particular to a system and method configured to monitor a kinetic state of the individual in order to assess sleep state.

Background of the Invention

Sleep disturbances can arise in many disorders, and for example are common in Parkinson's disease (PD). Fragmentation of sleep, characterized by repetitive short interruptions of sleep, is one important characteristic of sleep which can be assessed. Fragmented sleep may for example be caused by sleep apnoea, REM sleep disorders, restless legs, pain, nocturia, hallucinations and affective disorders. Sleep architecture, which refers to how an individual cycles through the stages of sleep, and sleep efficiency, being the percentage of time asleep, are also important characteristics of sleep.

Polysomnography (PSG), or a sleep study, seeks to obtain measures such as sleep efficiency, Arousal index, Apnoea Hypopnea Index and Periodic Limb Movements per hour to generate a report that takes into account these scores. PSG is the gold standard for sleep assessment but is heavily weighted to the assessment of apnoeas and has the disadvantage that it assesses sleep on a single night in conditions that are not typical for the patient. Moreover, sleep studies require the patient to spend a night sleeping in a clinical setting while being closely monitored, and are thus expensive, inconvenient and ill-suited to screening of large numbers of patients. In many countries or in remote areas, formal sleep studies are not even readily available.

Actigraphy has been attempted as a means to assess sleep in the home but has failed to accurately quantify sleep because it uses relatively unprocessed accelerometry and is thus overly affected by the limb movements of sleep.

A simple and effective means of detecting abnormal sleep would aid in identifying those who need further investigation.

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed before the priority date of each claim of this application.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

In this specification, a statement that an element may be "at least one of" a list of options is to be understood that the element may be any one of the listed options, or may be any combination of two or more of the listed options.

The article by Jennifer Girschik et al. "Validation of Self-Reported Sleep Against Actigraphy", Journal of Epidemiology, vol. 22, no. 5, 28 July 2012 (2012-07-28), pages 462-468 is directed to validating sleep assessment questionnaires against objective measures of sleep as assessed using actigraphy in a population of Western Australian women.

The article by Stavitsky et al. "Sleep in Parkinson's disease: A comparison of actigraphy and subjective measures", Parkinsonism and related disorders, ELSEVIER SCIENCE, OXFORD, GB, vol. 16, no. 4, 1 May 2010, pages 280-283, relates to assessment of sleep state of Parkinsonian subjects.

Summary of the Invention

According to a first aspect the present invention provides a method of assessing sleep state of an individual, as defined in appended independent claim 1.

According to a second aspect the present invention provides a system for assessing sleep state of an individual, the system being defined in appended independent claim 12.

According to a further aspect the present invention provides a non-transitory computer readable medium for assessing sleep state of an individual, as defined in appended independent claim 13.

Some embodiments of the invention may thus provide for measurement of night time sleep using an accelerometry based system suitable for use in a non-clinical setting such as the individual's home. Embodiments of the invention may thus provide a simple means of differentiating between normal and abnormal sleep, including abnormal sleep which is not caused by sleep apnoea.

Some embodiments may be applied to assess sleep state of Parkinsonian subjects.

Some embodiments may be applied to assess sleep state of non-Parkinsonian subjects.

In some embodiments, the sleep score may further be generated by summing or otherwise combining 2 or more of a set of sleep-related variables derived from the accelerometer data. For example, the sleep related variables may include a variable reflecting the individual's attempts at being active, such as a "percent of time active" (PTA) variable.

In some embodiments, the sleep related variables may include a variable reflecting the individual's inactivity while awake, such as a "percent of time inactive" (PTIn) variable.

In some embodiments, the sleep related variables may include a variable reflecting the individual's immobility while asleep, such as a "percent of time immobile" (PTI) variable.

In some embodiments, the sleep related variables may include a variable reflecting the individual's Sleep Duration.

In some embodiments, the sleep related variables may include a variable reflecting the individual's sleep fragment length, such as a "mean fragment length" (MFL) variable.

In some embodiments, the sleep related variables may include a variable reflecting the individual's Sleep Quality, such as a variable reflecting a proportion of time in a night period in which the individual was very immobile.

In some embodiments, combining 2 or more of a set of sleep-related variables derived from the accelerometer data may comprise the use of weights and combinatorial algorithms, the weights and algorithms being determined by a machine learning algorithm or the like configured to optimise selectivity and/or sensitivity of assessing a chosen condition.

According to the invention, the sleep score is produced only in respect of data obtained during a period of attempted sleep. The period of attempted sleep may be predefined, for example being preprogrammed into the device by a physician or technician. Alternatively commencement and/or conclusion of the period of attempted sleep may be partly or wholly defined by the individual in substantially real-time, such as by the individual making a user entry at the time of going to bed and/or getting out of bed. The user entry may be facilitated by any suitable user entry device, such as for example an app running on a tablet or smartphone or the like.

Brief Description of the Drawings

An example of the invention will now be described with reference to the accompanying drawings, in which:

Figures 1-3 illustrate a means for detection of kinetic state in accordance with an embodiment of the invention;
Figures 4-6 illustrate the efficacy of the described approach.
Figures 7A-7E illustrate sleep state of a control subject;
Figures 8A-8C illustrate sleep state of another control subject;
Figures 9A-9C illustrate sleep state of a person with Parkinson's;
Figures 10A-10C illustrate sleep state of another person with Parkinson's;
Figures 11A-11H illustrate the relative statistical importance of sleep state variables in differentiating differing sleep states;
Figures 12A-12I illustrate the relative statistical importance of sleep state variables, and sleep state scores derived therefrom, in differentiating differing sleep states;
Figures 13A-13I illustrate the correlation of sleep state variables, and sleep state scores derived therefrom, to a clinical standard; and
Figures 14A-14I illustrate the relationship between each variable and the PSG score.

Description of the Preferred Embodiments

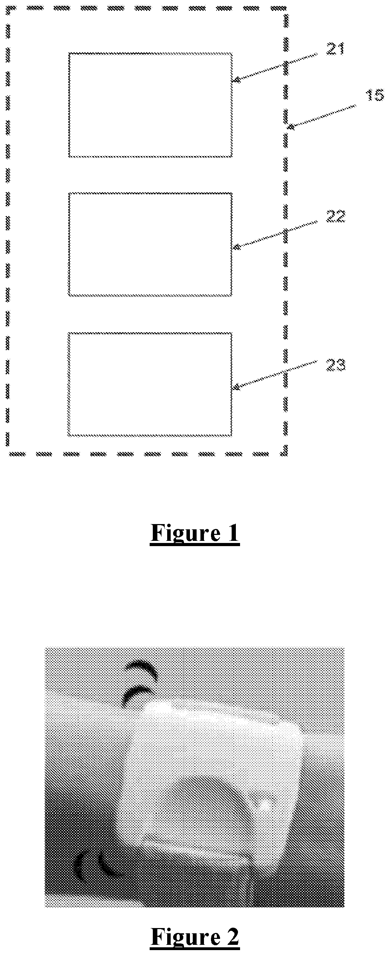

Figure 1 is a diagrammatic view of a device 15 for detection of kinetic state during an attempted sleep period of an individual, in accordance with an embodiment of the invention. The device 15 is wrist mounted which the present inventors have recognised provides a sufficiently accurate representation of the kinetic state of the whole body. The device 15 comprises three elements for obtaining movement data of a limb of a person. The device 15 comprises a motion monitor 21 in the form of an accelerometer, a data store 22 for recording the data, and an output means 23 for outputting movement data.

The device 15 is a light weight device which is intended to be worn on the wrist of the person as shown in Figure 2. The device is mounted on an elastic wrist band so as to be firmly supported enough that it does not wobble on the arm and therefore does not exaggerate accelerations. The device is configured to rise away from the person's wrist by a minimal amount, or not at all, so as to minimise exaggeration of movements. The device may be on a wrist band secured by a buckle, whereby the act of unbuckling and removing the device breaks a circuit and informs the logger that the device is not being worn.

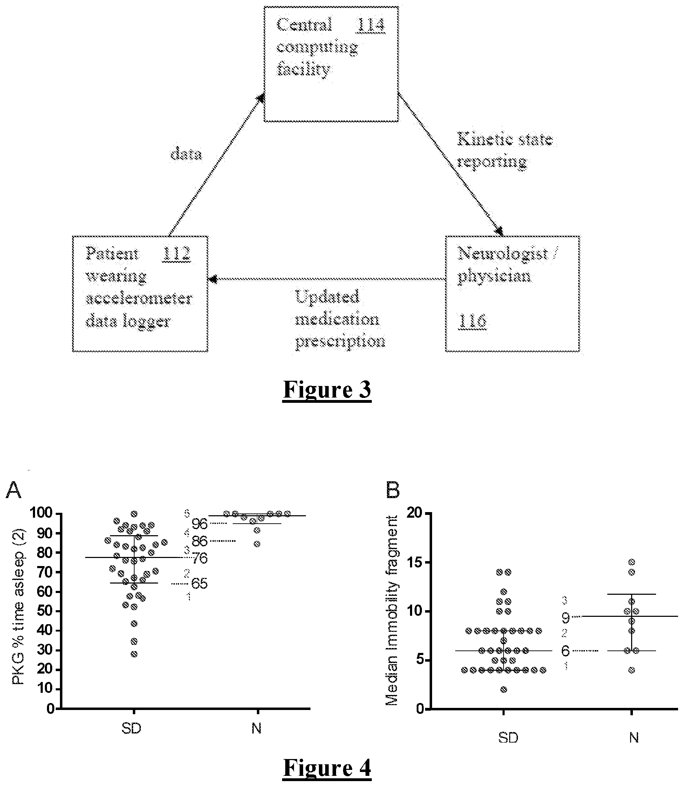

The user preferably wears the device throughout the night or throughout an attempted sleep period of interest. This allows the device to record kinetic activity of the individual for the sleep period. The accelerometer 21 records acceleration in three axes X, Y, Z over the bandwidth 0 - 10Hz, and stores the three channels of data in memory on-board the device. This device has sufficient storage to allow data to be stored on the device for a recording period of up to 12 hours, more preferably 10 days, after which the device can be provided to an administrator for the data to be downloaded and analysed. Additionally, in this embodiment, when the device is removed after the recording period, the device is configured to transfer the data to an associated device which then transmits the data via wireless broadband to analysis servers at a central facility (114 in Figure 3).

Figure 3 illustrates kinetic state monitoring and reporting in accordance with one embodiment of the invention. A user 112 is wearing the device of Figures 1 & 2. The device 15 logs accelerometer data and communicates it to a central computing facility 114. The computing facility 114 analyses the data using an algorithm (discussed further below), to obtain a time series of scores for the sleep state of the person 112. These scores are reported to a sleep physician 116 in a format which can be rapidly interpreted by the sleep physician to ensure efficient use of the physician's time. Physician 116 then interprets the sleep state report and implements or updates a treatment of the user 112 as required.

The accelerometer 21 measures acceleration using a uniaxial accelerometer with a measurement range of +/- 4g over a frequency range of 0 to 10 Hz. Alternatively a triaxial accelerometer can be used to provide greater sensitivity.

In this embodiment algorithms are applied to the obtained data by a central computing facility 114 in order to generate an assessment of a sleep state of the individual, referred to in the following as a PKG measure or score.

METHOD

In a first embodiment of the invention, described in relation to Figures 4 to 6, we performed simultaneous Polysomnography (PSG) and PKG measures in 45 subjects, 10 of whom had normal sleep. The PKG scores "periods of immobility" of at least 2 minutes and we used this to develop, amongst other measures, surrogates for SE (percent of attempted sleep time in which the patient was immobile) and fragmentation (the median length of each period of continuous immobility). These are called %time asleep (%TA) and median time immobile (MTI) respectively. We used these to develop a score that clearly differentiated between normal and abnormal sleepers as determined by the PSG report.

We then applied this PKG score to 24 age matched subjects without PD and 35 people with PD (PwP) who wore the PKG for 6 nights and responded to various questionnaires including Parkinson's Disease Sleep Scale 2 (PDSS-2). A further 45 PKG subjects were also analysed but without questionnaire.

RESULTS

The PKG score combining the %TA and the MTI predicted normal or abnormal sleep (according to the PSG) with 100% selectivity and sensitivity. In the 24 subjects without PD only 2 had abnormal sleep according to the PKG and one of these gave a history of restless legs. Amongst the PD subjects 28% had normal sleep according to the PKG criteria and in those interviewed, PKG values had a good correlation (r2 = 0.49) with the PDSS2 scale.

CONCLUSIONS

The PKG score appears to provide a simple means of detecting normal and abnormal sleep in PD. This is based on a small PSG sample.

The above example is now described in further detail. The sources of patients studied were as follows: 36 from Monash (Victoria, Australia) sleep lab and 9 from an epilepsy study (none of whom were thought to have a sleep disorder.

Normal (N): 8 of the epilepsy patients and two of the Monash sleep patients were reported as having normal sleep. Sleep Disordered (SD): See Table 2 for PSG diagnosis (col 3), scores from PSG (Col 4-7) and our classification (Col 2), which was based on the PSG diagnosis as shown in Table 1.

Table 1:

| PSG Δ | Controls | Mild -I | Mild plus | mild-mod | Mod | Severe minus | Severe |
|-------|----------|---------|-----------|----------|-----|--------------|--------|
| Score | 0 | 1 | 2 | 3 | 4 | 5 | 6 |

Table 2 (note, spread over 2 pages):

| 1) PKG Score | 2) PSG Score | 3) Conclusion | 4) Sleep efficiency | 5) PLM | 6) AI | 7) AHI |
|---|---|---|---|---|---|---|
| 8 | 0 | Unremarkable study | 82.3 | 3.3 | 11.9 | 0.7 |
| 8 | 0 | No significant sleep disordered breathing | 85.9 | 0 | 1.8 | 0.7 |
| 8 | 0 | There is no significant sleep disordered breathing MONASH | 93.2 | 6.8 | 12.1 | 2.3 |
| 6 | 0 | Normal sleep MONASH* | 75.4 | 7.7 | 25.1 | 0.5 |
| 5 | 0 | Normal study | 84.9 | 0 | 19.1 | 0.5 |
| 7 | 0 | Unremarkable study. Periodic leg movements were present and infrequently associated with cortical arousal | 90.4 | 21.8 | 17.5 | 1.5 |
| 8 | 0 | No significant sleep disordered breathing | 88.5 | 27.5 | 11.2 | 1.5 |
| 8 | 0 | no significant sleep disordered breathing | 84.3 | 0.2 | 18 | 0.8 |
| 7 | 0 | Unremarkable study. PLM not significant. | 95.2 | 12.5 | 23.3 | 1 |
| 6 | 0 | no significant sleep disordered breathing | 76 | 11.9 | 21.6 | 5.2 |
| 3 | 1 | mild REM predominant sleep disordered breathing with mild arterial oxygen desaturations in REM and stable SpO2 in NREM EPILEPSY STUDY | 82.5 | 7.1 | 23 | 30 |
| 4 | 1 | Mild REM based OSA | 89.5 | 0 | 6.6 | 6.1 |
| 4 | 1 | Normal? | 60.9 | 0 | 6.8 | 0.8 |
| 4 | 1 | Mild OSA | 88.4 | 9.9 | 12.9 | 6.1 |
| 2 | 1 | Adequate CPAP | 60.1 | 7.4 | 7.4 | 8.6 |
| 3 | 1 | Mild OSA | 57.3 | 10.7 | 22.1 | 6.1 |
| 2 | 1 | Normal study with high sleep tendency | 87 | 0 | 10.9 | 0.1 |
| 2 | 1 | No OSA, no narcolepsy, fragmented sleep | 85.6 | 2.4 | 25.4 | 1.4 |
| 3 | 1 | Mild OSA | 84.6 | 0 | 11.6 | 6.6 |
| 2 | 2 | Mild OSA, poor sleep efficiency | 57 | 1.3 | 14.2 | 5.6 |
| 3 | 2 | Baseline 02 sats 92 fell to 89% when asleep | 77.9 | 0 | 11.8 | 1.6 |
| 4 | 2 | Fragmented sleep | 81.5 | 0 | 8.9 | 0.2 |
| 3 | 2 | Mild OSA, fragmented sleep | 71.5 | 0.8 | 13.3 | 9.8 |
| 3 | 2 | Mild OSA, clusters of PLM | 78.3 | 13.3 | 18.2 | 9.7 |
| 4 | 2 | Mean reduced sleep latency- severe sleepiness | 91.3 | 8.1 | 21.8 | 4.1 |
| 2 | 2 | OSA, fragmentation | 68 | 0 | 24 | 38.8 |
| 2 | 3 | Mild-Mod OSA | 86.4 | 2.3 | 22.7 | 22.7 |
| 2 | 3 | Mod OSA | 85.6 | 0 | 25.6 | 19.2 |
| 2 | 4 | Mod OSA | 69.1 | 21.2 | 21.6 | 6.5 |
| 4 | 4 | Mod OSA | 84.1 | 0 | 29.5 | 22.1 |
| 3 | 4 | Mod OSA | 86.5 | 4.3 | 36.8 | 21.4 |
| 3 | 4 | Mod OSA | 85.7 | 0 | 26.2 | 20.2 |
| 2 | 4 | Mod-Severe OSA | 87 | 58.3 | 13.8 | 26.9 |
| 3 | 5 | Severe supine OSA (mild lateral) | 90.6 | 0 | 62.9 | 58.1 |
| 4 | 5 | Mod OSA, fragmented sleep | 91.4 | 12.2 | 37.6 | 19.6 |
| 2 | 5 | New CPAP levels prescribed | 83.1 | 43.9 | 34.2 | 6.6 |
| 3 | 5 | Mod-Severe OSA when supine | 85 | 24.2 | 12.6 | 13.2 |
| 2 | 6 | Severe OSA | 67.1 | 0 | 5.8 | 18.6 |
| 3 | 6 | Severe OSA | 72.7 | 0 | 14.9 | 33.8 |
| 3 | 6 | Poor sleep | 66.6 | 32.6 | 15.6 | 3.6 |
| 3 | 6 | Severe OSA | 62.5 | 4.7 | 27.7 | 48.8 |

(continued)

| 1) PKG Score | 2) PSG Score | 3) Conclusion | 4) Sleep efficiency | 5) PLM | 6) AI | 7) AHI |
|---|---|---|---|---|---|---|
| 4 | 6 | Severe OSA | 73.8 | 0 | 40.5 | 32.8 |
| 3 | 6 | Severe OSA | 82.5 | 0 | 26.9 | 52.9 |
| 2 | 6 | Severe OSA | 91.9 | 0 | 26.3 | 57.6 |
| 2 | 6 | Severe OSA, very abnormal sleep | 48.3 | 25.4 | 45.3 | 38.7 |
| 2 | 6 | Severe OSA, fragmented sleep | 86.4 | 0 | 35.3 | 41.3 |
| 3 | 6 | Severe OSA | 91.2 | 0 | 44.1 | 89.4 |

Column 1 of Table 2 contains the score as estimated from the PKG measures in accordance with the present embodiment of the invention. Two values were used to produce a PKG score: The Percent Time Asleep, which is a measure of the proportion of time immobile over the period in which sleep was attempted (akin to sleep efficiency) and the median length (duration) of each period of immobility making up the sleep (akin to a measure of fragmentation). A number of other markers were examined but these two provided a degree of difference between SD and N. Percent Time Asleep was then scored with a level of severity from 1-5 (with 1 being most affected and 5 being normal) based on the median, 75th and 90th percentile of normals (for Percent Time Asleep) as well as the 75th percentile of SD. Median Duration of immobility was then scored with a level of severity from 1-3 (with 1 being most affected and 3 being normal) based on the median and 75th of normal.

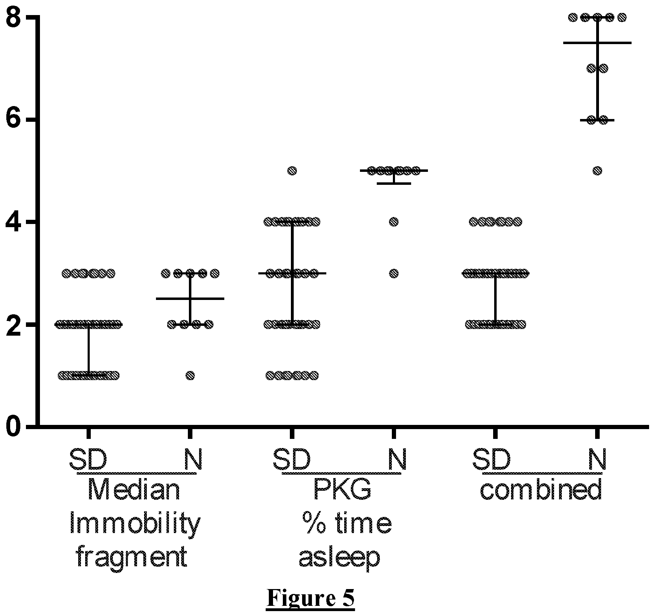

The Scores for the PKG and the PSG were then compared (Figure 4). A score of 1.5 for immobility gave a high sensitivity (90%) for finding normal subjects but with very poor selectivity (38%). A score of 3.5 for % time asleep gave a sensitivity (90%) for finding normal subjects with an improved selectivity (70%). Adding the two (as shown in Figure 5) however gave complete separation between normal and abnormal subjects when the used threshold score was 4.5. This suggest that the combined PKG score would be a good screening tool to detect abnormal sleep if a score of <5 or even <6 was used. Combining the scores can be simple addition of the scores as shown in Figure 5 or in other embodiments could be any other suitable linear or more complex non-linear combination of the %TA and MTI scores which elicits improved sensitivity and selectivity.

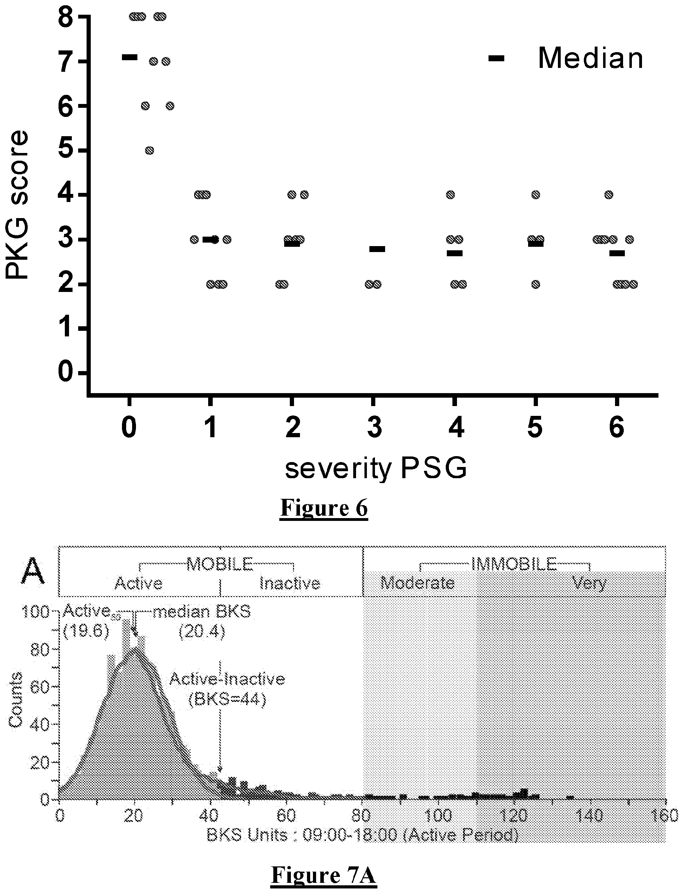

The next step was to compare the PKG score with the PSG (Table 1, Figure 6). This further confirms that the PKG score is helpful for sorting into "normal" and "abnormal" sleep but not in grading severity further in terms of matching severity by PSG. Note that the sleep abnormalities in the PSG were most severe for OSA and these are not necessarily the reason for having abnormalities of sleep in PD.

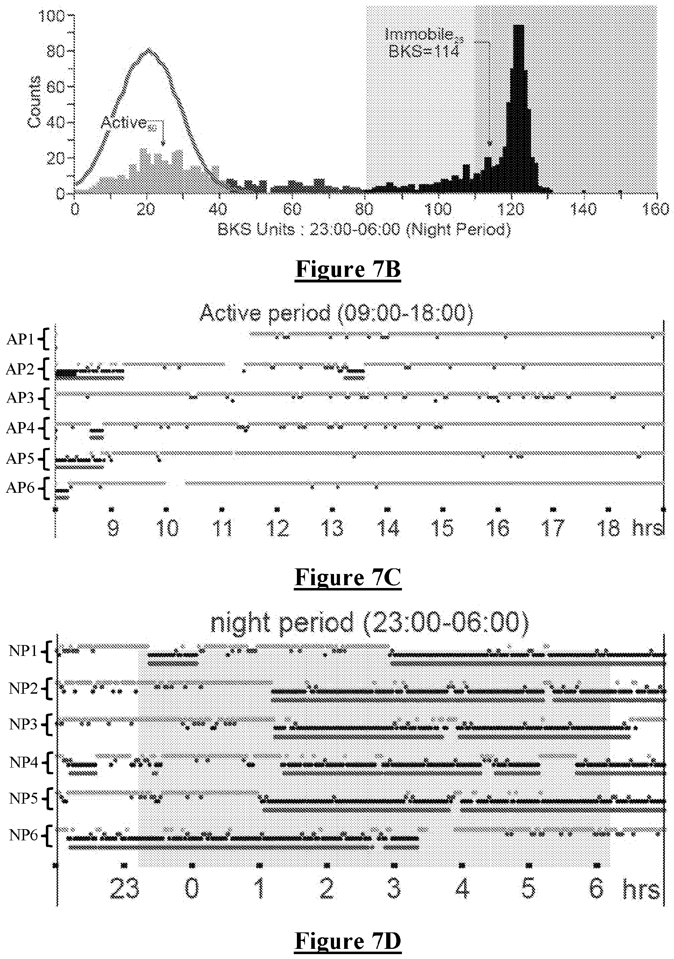

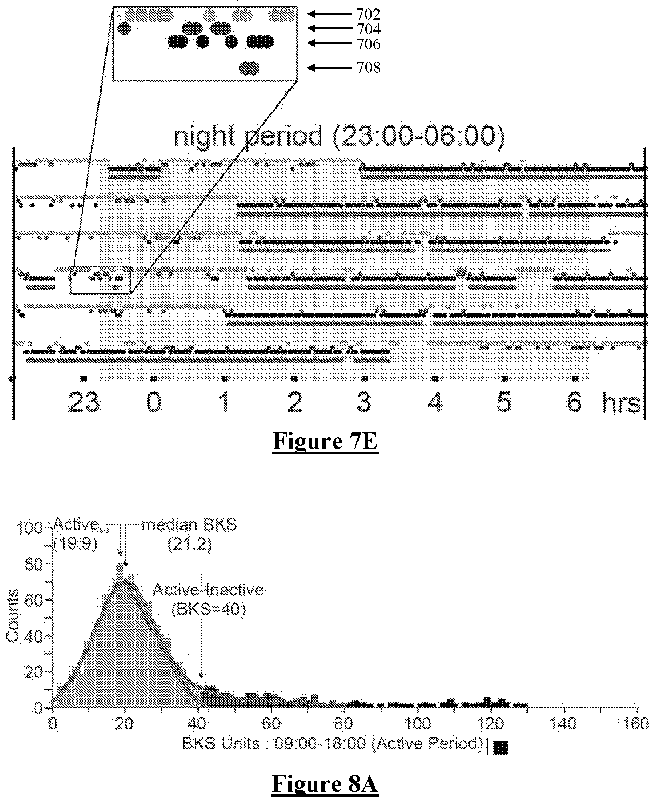

Figures 7 to 14 illustrate further embodiments of the present invention. In these embodiments, normal ranges for the respective scores were obtained from a cohort of 155 subjects aged 60 years or more without known neurodegenerative disorders. The comparison group was 72 PD subjects.

The various scores assessed, and their derivation, is as follows. The time period of data recording was divided into periods based on the time of day, as follows. An Active Period (AP) during the hours 09:00-18:00, chosen because most subjects are active and pursuing their usual daily activity in this period. A Night Period (NP) was examined for quality of nocturnal sleep. A Rest Period (RP) during the hours 08:00-23:00 was chosen to represent a period when most people are sedentary.

DEFINITIONS OF MOVEMENTS

A dyskinesia score (DKS, or DK score) is calculated every two minutes throughout the period of time that the logger is worn. In the presently described embodiments the DKS is calculated in accordance with the teachings of International Patent Publication Number WO 2009/149520, however in alternative embodiments the DKS may be determined in any suitable alternative manner.

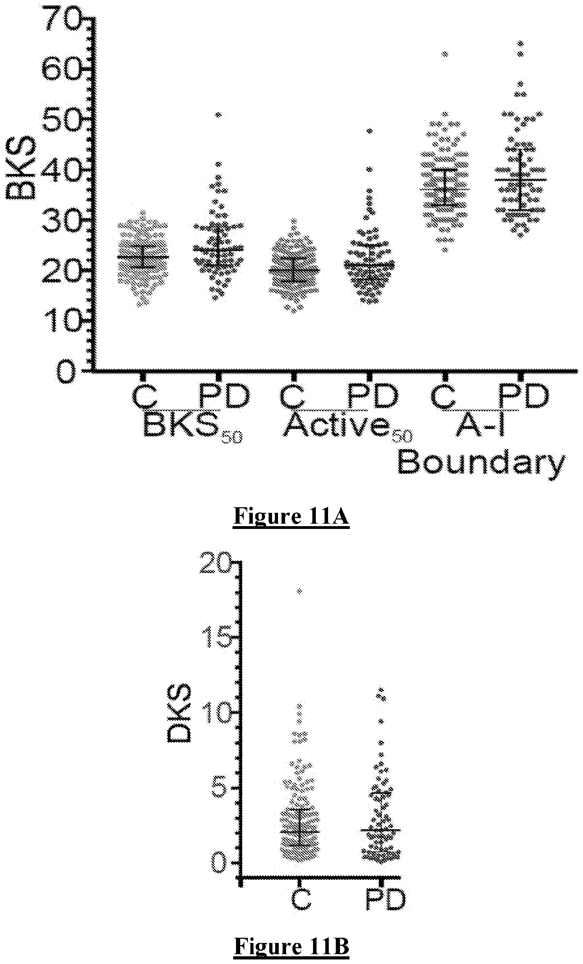

Median DKS. The median value of the DK scores from the AP. The Median DKS correlates with the Abnormal Involuntary Movement Score assessed at the time of donning the PKG logger. Figure 11b shows the Distribution of the median DKS for the control group and the PD Group. Table 3 below sets out the values observed for DKS in each group, in particular being the minimum observed DKS value, the 10th, 25th, 75th and 90th percentile values of DKS, the Median DKS, and the maximum observed DKS value. It is to be noted that DKS may be measured on any suitable scale, and may be assessed by reference to any suitable division of percentile bands. For example alternative embodiments of the present invention may use four percentile bands in the manner described in the above-referenced WO 2009/149520, specifically DK I (0-50th percentile of normal) DK II (50th - 75th percentile of normal), DK III (75th - 90th percentile of normal) and DK IV (>90th percentile of normal).

Table 3: Scores that contribute to AP Assessment

| | | PERCENTILE | | | | | | |
|---|---|---|---|---|---|---|---|---|
| | | Min | 10% | 25% | Median | 75% | 90% | Max |
| DKS | C | 0.2 | 0.66 | 1.2 | 2.1 | 3.6 | 6.22 | 18.1 |
| | PD | 0.1 | 0.33 | 0.725 | 2.2 | 4.65 | 6.54 | 11.5 |
| BKS_ | C | 13.2 | 17.82 | 20.5 | 22.6 | 24.9 | 28.04 | 31.4 |
| | PD | 14.5 | 18.15 | 20.83 | 24 | 28.63 | 35.14 | 50.9 |
| $ACTIVE_{50}$ | C | 12 | 15.96 | 17.8 | 19.9 | 22.4 | 24.32 | 29.8 |
| | PD | 13.8 | 15.7 | 18.2 | 21.05 | 24.98 | 31.13 | 47.7 |
| $Boundary_{A-I}$ | C | 24 | 30 | 33 | 36 | 40 | 44.4 | 63 |
| | PD | 27 | 30 | 32 | 38 | 44 | 51 | 65 |
| PTA | C | 49.1 | 65.98 | 71.2 | 78.5 | 84.8 | 91.04 | 95.4 |
| | PD | 42.6 | 56.09 | 68.03 | 76.15 | 84.28 | 89.95 | 92.2 |
| PTIn | C | 1.4 | 7.52 | 10.6 | 16 | 22.2 | 28.24 | 41.6 |
| | PD | 1.8 | 6.26 | 9.075 | 15.85 | 24.15 | 29.14 | 43.9 |
| PTI | C | 0.1 | 1.2 | 2.1 | 4 | 6.9 | 10.24 | 18.9 |
| | PD | 0.1 | 1.1 | 3.525 | 6.6 | 11.13 | 16.78 | 31.5 |

A bradykinesia score (BKS, or BK score) is calculated every two minutes throughout the period of time that the logger is worn. In the presently described embodiments each BKS is calculated in accordance with the teachings of International Patent Publication Number WO 2009/149520, however in alternative embodiments the BKS may be determined in any suitable alternative manner. It is to be noted that, as for DKS, the BKS may be measured on any suitable scale, and may be assessed by reference to any suitable division of percentile bands. Over each period of analysis (e.g. AP or NP), the BKS can be examined as a frequency histogram of the values for BKS in the manner shown in Figures 7A, 7B, 8A, 8B, 9A, 9B, 10A and 10B. The present embodiment recognises that the BKS can be grouped into two super categories referred to herein as a Mobile category and an Immobile category, and that each in turn can be further divided into two subcategories, referred to herein as Active Mobile, Inactive Mobile, Moderate Immobile and Very Immobile, as shown in Figure 7A. See Figure 11A for the BKS distribution and Table 3 above for the values observed for BKS in each group.

In more detail, Figure 7A is a histogram of BKS units in a Control (non PD) subject from the Active Period (AP). Figure 7B is a histogram of BKS units in the same subject from the Night Period (NP). In Figures 7A and 7B, the x axis is the value of the BKS unit and the Y axis is the number of BKS units with that value. Each histogram shows the four types of BKS categories: Active (0 < BKS < 44), Inactive (44 < BKX < 80) and Immobile (80 < BKS), which is divided into a Moderate Immobile category (80 < BKS < 110) and a Very Immobile category (110 < BKS).

The $Active_{50}$ value is defined in this embodiment as being the median (and mode) of the Active BKS during the AP. The distribution of the BKS is shown in red in both histograms. It is noted that the distribution of Active BKS in the night period histogram of Figure 7B is similar to the day period histogram of Figure 7A. The median BKS of 20.4 for the subject of Figure 7 is similar to the $Active_{50}$ value of 19.6, as is generally the case for normal subjects.

In Figure 7A it is notable that during the AP, Inactive BKS are uncommon, as are Moderate Immobile BKS and very Immobile BKS values. In contrast, in Figure 7B it is notable that during the NP there is a marked peak of Very Immobile BKS values, with the histogram peak occurring at a BKS value of ~125. In Figure 7B the 25th percentile of BKS values in the Very Immobile range (referred to as the $Immobile_{25}$ value) has a value of 114.

Figure 7C is a raster plot of 6 six consecutive days denoted AP1 to AP6, showing data from the AP of each day. Each BKS value is shown as a light blue dot in the top row if the BKS is in the Active range (0-44), or as a dark blue dot in the second row if the BKS is in the Inactive range (44-80), or as a black dot in the third row if the BKS is in the Immobile range (BKS>80). A red dot is shown in the fourth row of each raster trace if at least four of the surrounding consecutive BKS values are >80. Each red dot thus indicates that the surrounding 7 consecutive BKS scores reflect the existence of a "sleep epoch". It is notable in Figure 7C that this subject was awake (ie not immobile) and active (most dots light blue) for most of the AP on each of the 6 days observed.

Figure 7D is a raster plot of six consecutive evenings, showing data from 22:00-07:00 but with NP shaded in light grey. Each BKS is coloured and positioned in one of four rows, using the same convention described above in relation to Figure 7C. It is notable in Figure 7D that the BKS data indicate that this subject was active (with blue dots in the top row) until about 01:00 during night periods NP2 - NP5, and was "asleep" until at least 07:00 during night periods NP1, NP2, NP4 and NP5. On NP6 this subject went to sleep about 3 hours earlier than the other nights and rose shortly after 03:00, which for example might be indicative of a shift worker.

Figure 7E provides an enlarged view of a portion of the raster plot of Figure 7D, illustrating the top row 702 of Active BKS values, the second row 704 of Inactive BKS values, the third row 706 of Immobile BKS values, and the fourth row 708 of sleep epoch data points.

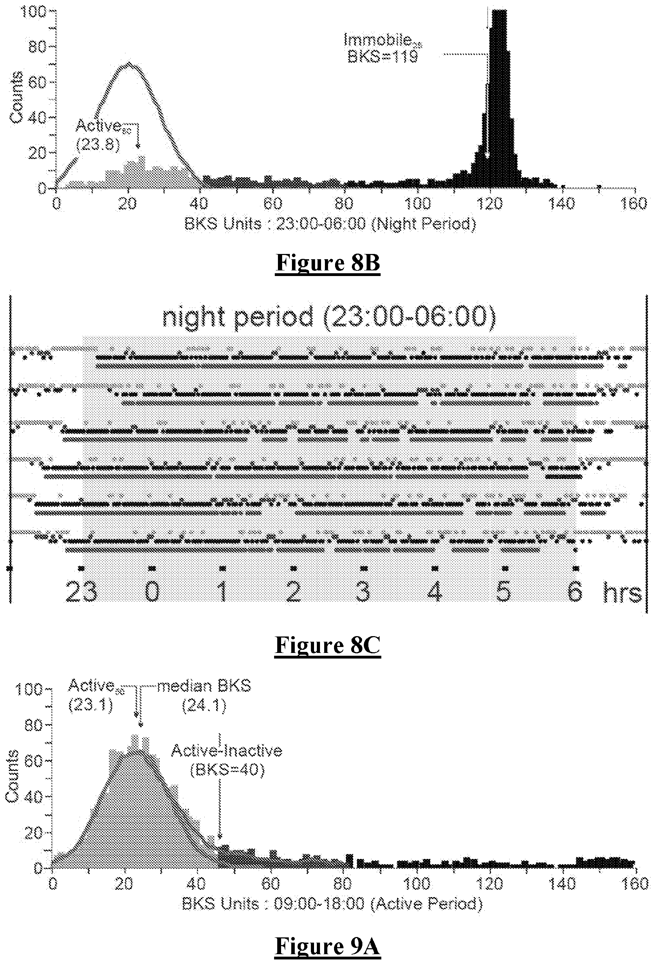

Figure 8 shows data obtained from another normal control subject, using the same plotting conventions as Figure 7. In particular, Figure 8A shows a histogram of BKS values from the second control subject during the AP (09:00 - 18:00), and Figure 8B shows the BKS values from the NP (23:00 - 06:00). Figure 8C shows that this person falls asleep most nights around 23:00 and awakes around 06:00 each morning and exhibits relatively normal sleep between those times.

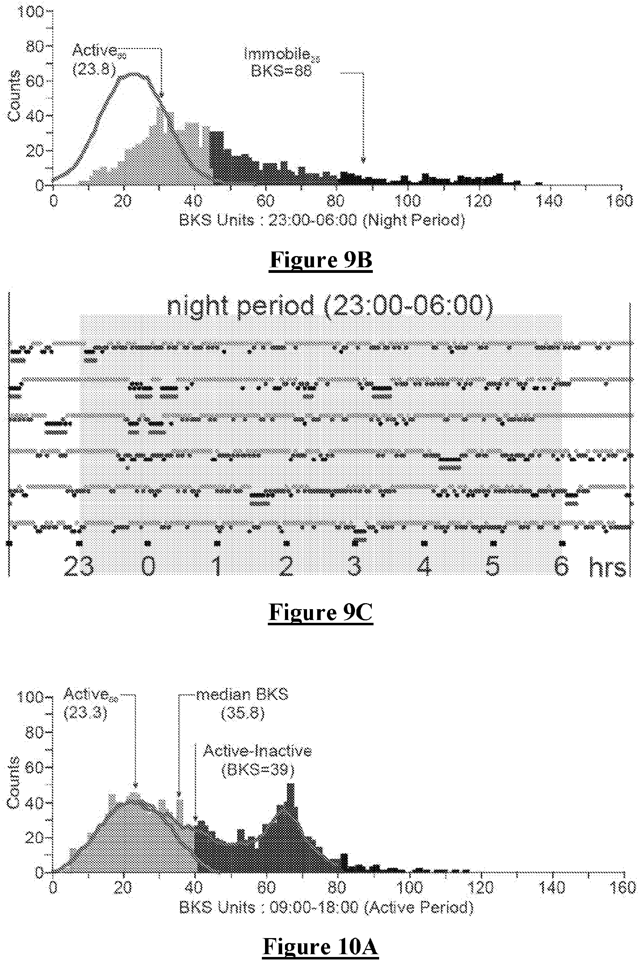

Figure 9 shows BKS data obtained from a Person with Parkinson's (PwP). Figure 9A shows that, during the AP, this person exhibits increased Immobile BKS measures. Moreover, Figure 9B shows that during the NP a markedly abnormal sleep pattern exists. This is revealed by very little BKS in either the very immobile or immobile range as compared to the controls of Figures 7B and 8B. The abnormal sleep is also evidenced in Figure 9B by way of the increased Inactive and active data throughout the record, as compared to the control subjects of Figures 7B & 8B. It is noted that the $Active_{50}$ is only modestly elevated in the PwP in Figures 9A & 9B, as compared to the $Active_{50}$ in Figures 7 and 8.

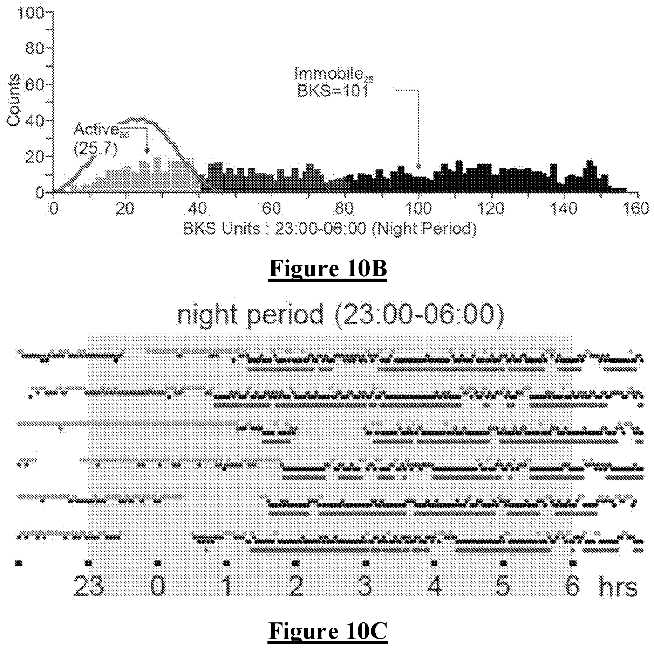

Figure 10 shows the data from another PwP. This subject exhibits a marked preponderance of Mobile Inactive BKS Values during the AP, even though there is little Immobility (i.e., little day time sleep). Figure 10C shows that the subject is late retiring, typically falling asleep around 01:00 - 01:30. Figure 10C further shows that this subject has reasonably long periods of "sleep" as shown by Sleep epochs in the fourth row of each raster plot. However, when Figure 10B is considered it can be determined that the Sleep Quality is poor as shown by a low $Immobile_{25}$ (of BKS=101) and relatively few occurrences of Very Immobile BKS values in Figure 10B as compared for example to the strong Very Immobile peaks in Figures 7B and 8B for normal subjects. Moreover, in Figure 10B there exists relatively increased Inactive BKS at night as compared to Figures 7B and 8B for normal subjects, and the Active BKS has a higher $Active_{50}$ (=25.7) than normal subjects, suggesting night time bradykinesia.

As shown in Figures 7A & 7B, in which BKS values are returned on a scale of 0-160, for BKS values greater than 80 the subject is defined as being Immobile. BKS > 80 are thus a surrogate marker for daytime sleep. When BKS during the NP are examined in healthy subjects (as in Figure 7B and Figure 8B), it is apparent that most Immobile BKS values are part of a Gaussian distribution with very high BKS (typically greater than 110) with a long left sided tail. It is also apparent that People with PD (PwP) are less likely to have this peak of high BKS (see Figure 9B and 10B) and have generally lower BKS. We have calculated $25^{th}$ percentile of all Immobile BKS values in the NP for each patient and the median of all these values from the 155 Control subjects was produced and called **$Immobile_{25}$** which was at BKS=111.

The Moderately Immobile range (MI) is when BKS is between 80-110. The Very Immobile (VI) range is when BKS =111 or greater. "Good sleepers" have a high proportion of Immobile BKS>110. See Discussion of "Sleep Quality" Below.

Day Time Immobility (PTI) is defined as the percentage of time during the AP with Immobility, and has been correlated with polysomnographic recordings of sleep in the daytime. Immobility during the AP is mainly in the MI range when present in normal subjects (Figure 7A, 7C and 8A) and in many patients (Figure 9A & 10A). Table 3 sets out the normal ranges for PTI as determined from the 155 control subjects and the 72 PD subjects.

BKS<80 are broadly defined as Mobile. Examination of the Mobile BKS (eg Figure 7A, 7B, 8A and 8B) suggests that there are two distributions within BKS <80: a Gaussian distribution typically less than 40-50 BKS and a separate distribution between 40 and 80. Principle Component Analyses (PCA) supported the conclusion that there were indeed two components with BKS<80. Figure 10A shows an extreme example of a subject clearly exhibiting the separate distribution of BKS in the 40-80 range, independently of and in addition to the Gaussian distribution of BKS <40. Accordingly, this is reflected by the division of Mobile BKS into Active Mobile and Inactive Mobile as shown in Figure 7A.

Active BKS are thus BKS measures which fall in the lower Gaussian Distribution. To quantify Active BKS it was therefore first necessary to extract this distribution from the broader data set. To do so, in this embodiment it was assumed that the slope or curve of the BKS values from BKS=0 to the peak (the mode of the distribution outlined by the red line in Figure 7A, 7B, 8A, 8B, 9A, 9B, 10A and 10B) represents the left half of the Gaussian distribution sought. This "left half" is then smoothed from BKS=0 to the mode BKS (shown as the red line in the three graphs in Figure 7) using an 11-point boxcar filter. This smoothed line is then 'reflected' around the mode to produce the full Gaussian distributed component of the graph (and is shown as the red bell shaped curve in Figures 7A, 7B, 8A, 8B, 9A, 9B, 10A and 10B).

It is to be appreciated that any suitable method of extracting the lower distribution may be used in accordance with other embodiments of the present invention. All BKS values enclosed by this curve represent Active BKS and the 50th percentile (and mode) of these values is referred to as the **Active$_{50}$**. Without intending to be limited by theory, it is proposed that this subset of the BKS data represents the BKS that are related to and arise from the subject's attempts at being active. The proportion of BKS within the Active Distribution during the AP is referred to as the Percent Time Active (PTA). The upper limit of the Active Distribution is typically about twice the Active$_{50}$ and is also the boundary between Active scores and those that lie between this limit and BKS=80 (Inactive Scores). This boundary between Active and Inactive is referred to herein as Boundary$_{A-I}$. It is to be appreciated that any suitable value may be selected or determined for Boundary$_{A-I}$. Figure 11A shows plots of the distribution of median BKS (BKS$_{50}$), Active$_{50}$, and the boundary between Active and Inactive BKS (A-I Boundary) in normal subjects (C) aged greater than 60 and PwP (PD).

Figure 11B shows plots of the distribution of median DKS (DKS$_{50}$) in normal subjects (C) aged greater than 60 and PwP (PD).

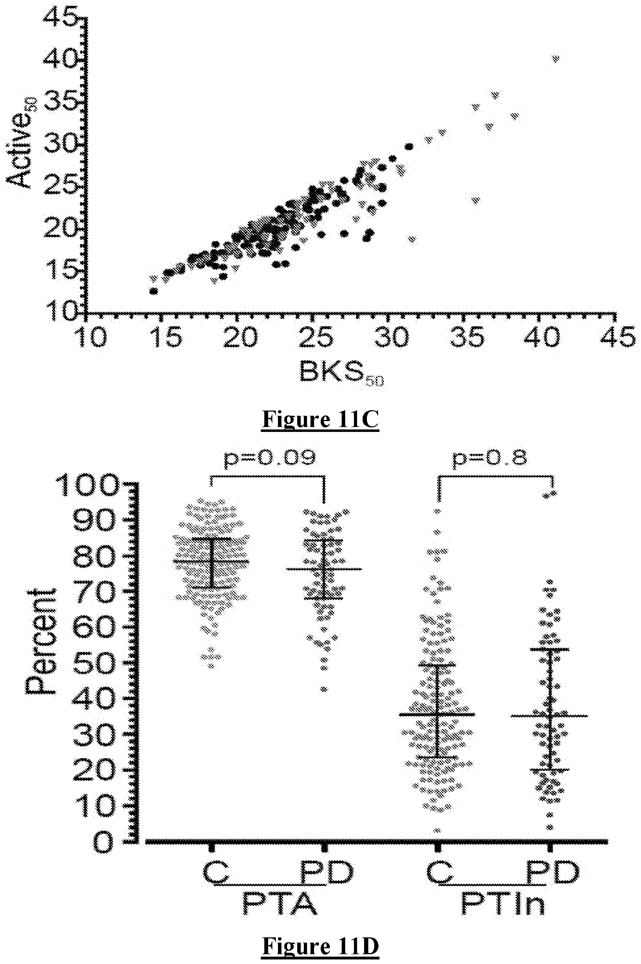

Figure 11C is a plot of the difference between median BKS (BKS$_{50}$) on the X axis and Active$_{50}$ on the Y axis showing these values for both Controls (black dots) and PwP (red triangles). In most cases there is a modest reduction in the Active$_{50}$ but on occasions the reduction is large with higher BKS (eg as shown in Figure 10).

Inactive. BKS values that lie between the Boundary$_{A-I}$ and BKS=80. It is assumed that these BKS indicate movement associated with sedentary behaviour and in particular somnolence. They are temporally more common at times when Immobility scores are present and also in the RP when TV and drowsiness often occur. Figure 10A shows a marked excess of BKS in the Inactivity range. The proportion of BKS within the Inactive Distribution during the AP is referred to as the Percent Time Inactive (PTIn) with control and patient values set out in table 3.

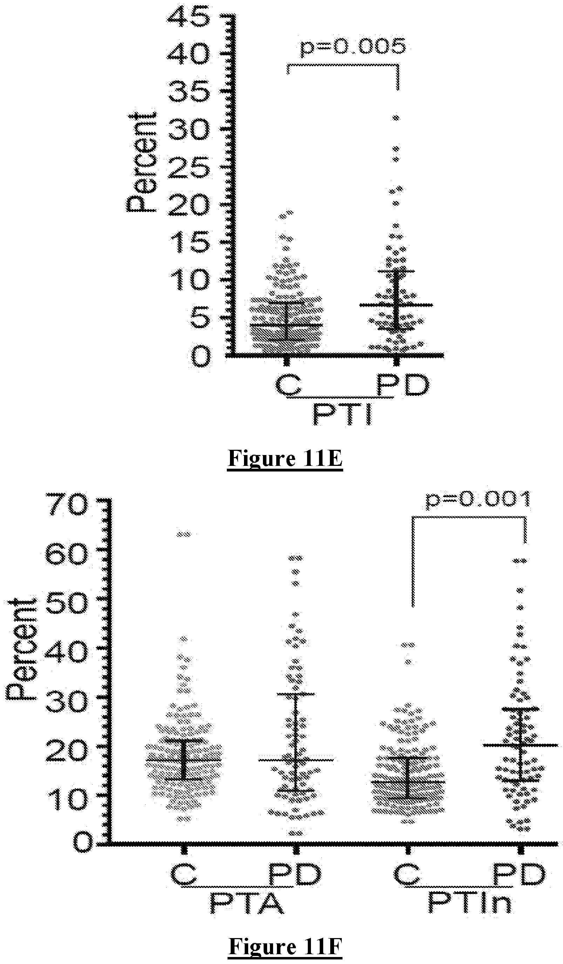

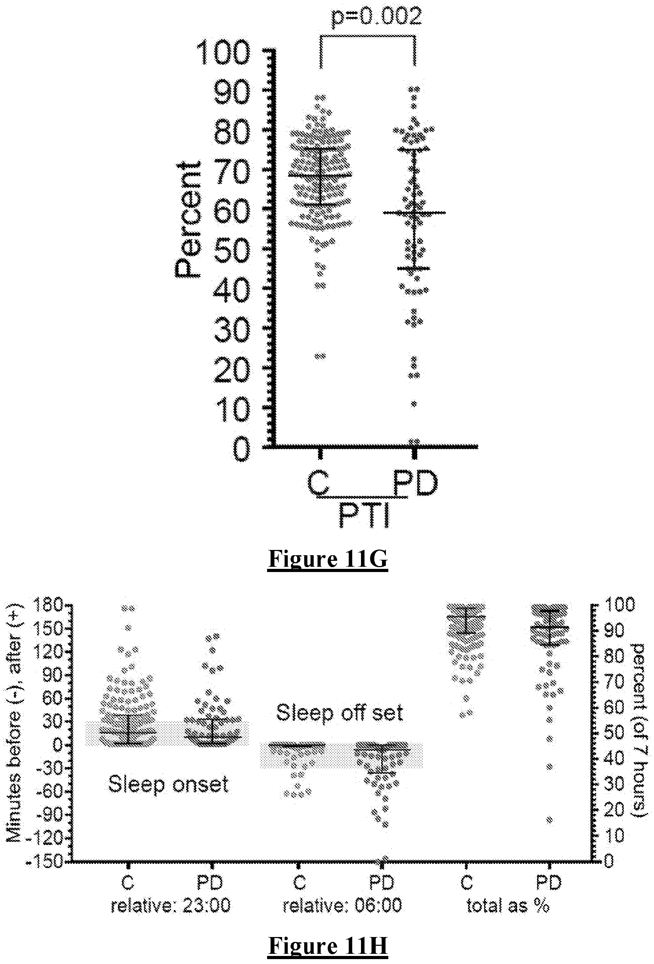

All BKS categories (Mobile (Active, Inactive) and Immobile (MI and VI) are used in all periods including AP, RP and NP and their percentage time in these categories varies according to which period is being examined (see Table 3 and Figure 11). Figures 11D, 11E, 11F and 11G show the PTA, PTIn and PTI in the AP and NP. As expected PTA is higher in the AP whereas the PTI is higher in the NP. PTI is significantly higher in the day time and lower at night in PwP, compared with controls.

NIGHT PERIOD AND SLEEP SCORES

In assessing sleep, we have used the units: BKS>80 or PTI and Sleep Epochs.

**PTI:** In the NP is, in effect, the proportion of time in the NP that the subject was immobile. This correlates with sleep in the day but may not be as good a correlation in the NP because people may move (BKS<80) during nocturnal sleep. In more detail, the range of BKS used in this embodiment extends from values of 1 to 150, and there is progressively less energy in the movement as the scores increase. While BKS scores from 80 to 150 do not reflect precisely zero movement, we define herein that the person has "moved" only if the BKS<80, and that for BKS > 80 there exists a range of immobility including both the Immobile and Very Immobile bands.

**Sleep Epoch:** To address the issue of movement during sleep, a sleep epoch was produced by taking 7 consecutive BKS values: if the BKS in 4 of the 7 values is >80 then we deem the central epoch as "sleep". We then "slide" the assessment forward in time by 1 BKS epoch and ask again if 4/7 are >80 to score the next BKS as "asleep" or "awake".

Factors that might be considered in assessing sleep include:
**Efficiency:** the extent to which a person slept, throughout the period in which sleep was attempted. This is achieved in the Polysomnography (PSG) lab by measuring time asleep during the period from lights OFF to lights ON. This is difficult at home or otherwise out of the clinical setting with the body worn device of the present invention, because we can only assess when sleep began and not the period over which sleep was attempted (ie in bed and trying to sleep). The choice of the NP being from 23:00 to 06:00 is made because ~75% of subjects were asleep within 30 mins of 23:00 and >90% slept till 06:00, as shown in Figure 11H. In particular, Figure 11H shows the time that control subjects and PwP retired relative to 23:00 or awoke relative to 06:00. Note that sleep onset before 23:00 or after 06:00 was not assessed. "Total as %" (right Y axis) refers to the time between first sleep (measured by a train of consecutive sleep epochs: either already asleep at 23:00 or first appearance after 23:00) and last sleep (either before or ending at 06:00) expressed as a percent of the 420 available minutes. Even though the resulting NP is less than the "standard 8 hours", most people are asleep over this period (Figure 11G) and so we assess the amount of "sleep" by reference to such a definition of NP by the following estimates.

In all figures bars show the median and interquartile range. These ranges are tabulated in Table 3.

**PTI:** This is the proportion of the NP in which BKS>80. While it broadly correlates inversely with time between Offset and onset of sleep, in control the PTI is ~ 25% lower. The PTI is in effect a measure of sleep efficiency

**Sleep Duration.** This is the sum of the number of sleep epochs in the NP (multiplied by two to be expressed in minutes). It correlates with PTI with an $r^2$=0.81.

**PTIn:** Subjects who have made movements in their sleep or are awake but attempting sleep, may have BKS<80 and in the Inactive range for that subject.

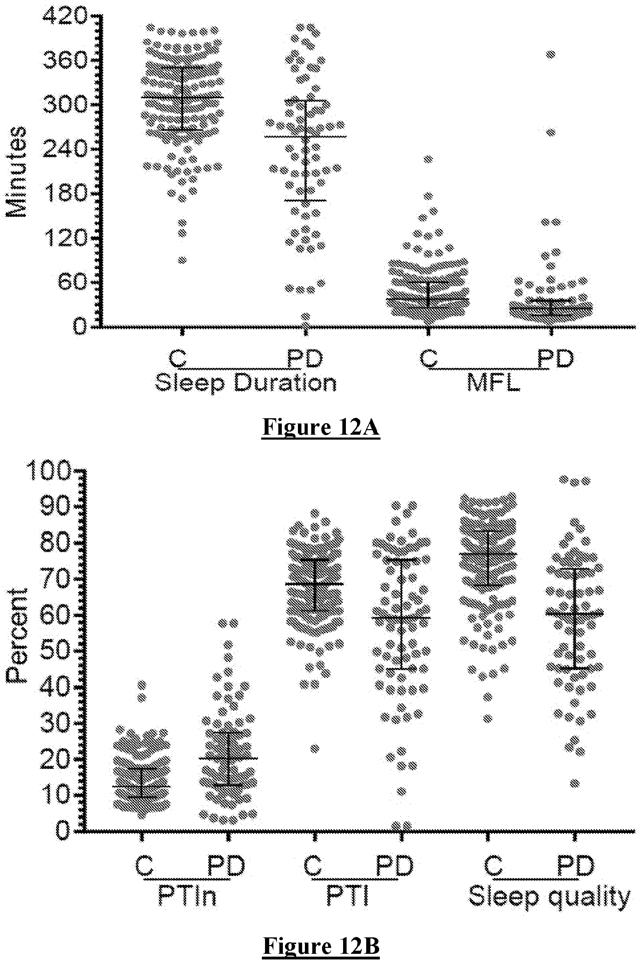

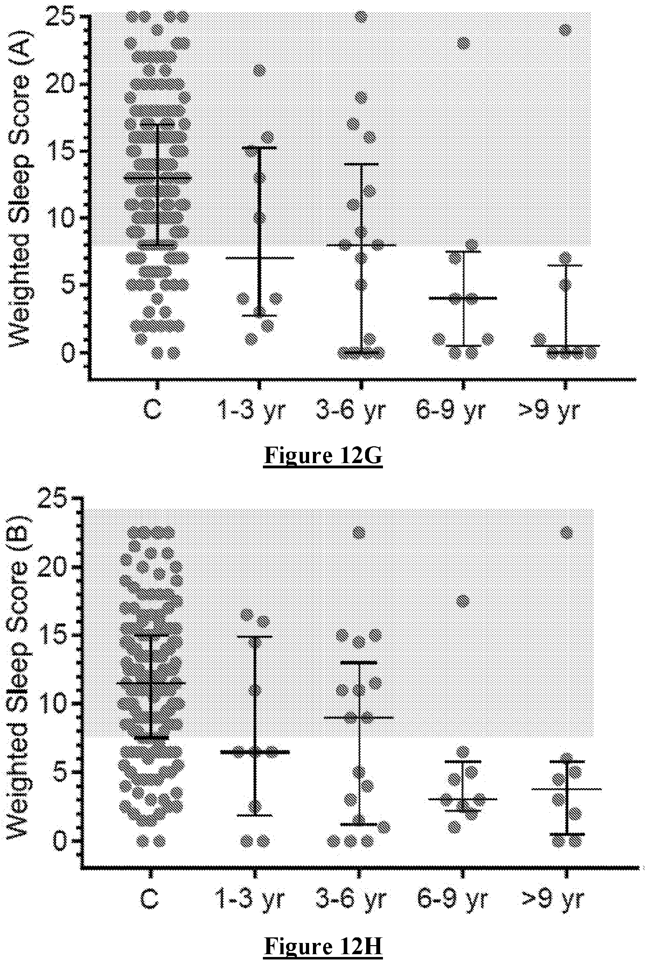

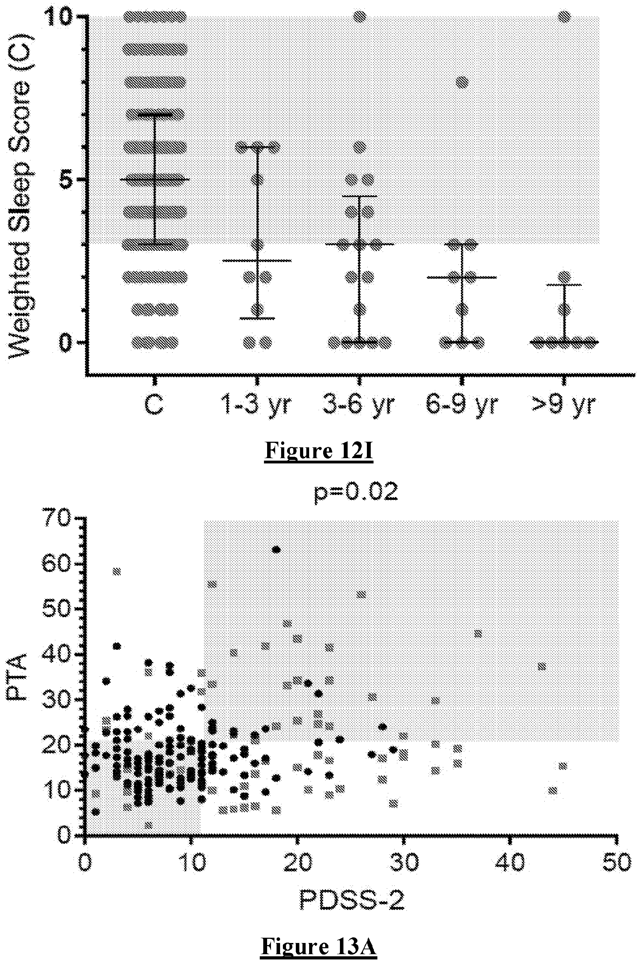

**Fragmentation:** If a person is immobile from sleep for a period - say 20 minutes - then there will be 10 consecutives 2 minute Sleep Epochs. Such a stretch of consecutive Sleep Epochs is termed a **sleep fragment.** We postulate subjects who have frequent micro-arousals and periodic limb movements (PLM) are likely to have shorter fragments. In most subjects, the distribution of fragment length is markedly hyperbolic and even though there is a high proportion of short fragments, most of the sleep (immobility) resulting from a small number of long fragments. Thus a measure of fragmentation would be to estimate the proportion of sleep (immobility) resulting from fragments greater than a certain length. To estimate this, we measured the **median fragment length** (MFL). Control and PD subject values are shown in Figure 12A.

**Architecture:** Sleep Studies suggest that the full sleep architecture requires longer sleep segments and that micro-arousals and periodic limb movements are less frequent during deeper stages of sleep. This suggest that the presence of a proportion of sleep with less movement may reflect better quality sleep architecture. The $Immobile_{25}$ (as defined in the preceding) for each Control subject was found, and the median $Immobile_{25}$ of all subjects was then calculated (BKS=111). This was used as the boundary between MI (moderate immobility) and VI (very immobile). We then estimated the proportion of time in the NP, that each subject was Very Immobile (VI) and called this **"Sleep Quality".**

**Time Awake.** This is related to a number of factors. This includes those related to poor sleep hygiene (late to bed, early rising): factors related to sleep disruption (pain, bladder control etc.): factors related to mood or disrupted sleep regulation (e.g. early awakening from depression). The premise here is that frank awakening will be captured in part by Active BKS (PTA, as described above) rather than PTIn. Arguably "Time Awake" will be inversely related to Sleep Efficiency.

SLEEP SCORE.

Six variables have been described above (PTA, PTI, PTIn, Sleep Duration, MFL, Sleep Quality) but there may be a degree of overlap between them as descriptors of "good sleep". Furthermore, each type of sleep disorder is likely to manifest in its own way in such kinetic observations and so a different set of variables might be required to accurately assess different sleep disorders. Thus, the present invention recognises that combining a set of these variables with variable weightings into a single score might better describe disordered sleep, and moreover that different variable weightings can be used to assess different disorders. We use the following steps.

*Step 1. For a particular individual, give each variable a score ranging from 0-5.* This is because each variable has a different range (some percentages (0-100) and others in minutes and less than 30 units) and distribution, so they must be normalised if they are to be summed. To achieve this the 10th, 25th, 50th, 75th and 90th percentile of each variable were found and these were used as a scoring system. A score from 0-5 was given according to Table 4. Note Table 4 provides two inverse options for this conversion, depending on whether the assessment should return higher scores to indicate better sleep, or lower scores to indicate better sleep.

Table 4. How sleep variables are traNsferrred to a common scoring system

| Percentile range | High score =good sleep | Low score = good sleep |
|---|---|---|
| 0-10 | 0 | 5 |
| 10-25 | 1 | 4 |
| 25-50 | 2 | 3 |
| 50-75 | 3 | 2 |
| 75-90 | 4 | 1 |
| 90-100 | 5 | 0 |

*Step 2. Sum and weight each normalised variable.* A Sleep Score for a particular condition (eg PD) could be produced according to the following formula:

$$\text{Sleep Score (PD)} = a \times \text{PTA} + b \times \text{PTI} + c \times, \text{PTIn} + d \times \text{Sleep Duration} + e \times \text{MFL} + f \times \text{Sleep Quality}$$

where *a, b, c, d, e* and *f* are weightings that might range from 0 (no weight) to some value greater than 1 (to increase the weight). These weights might be determined by inspection, by trial and error or by using machine learning.

*Step 3. Determine the weightings for a particular condition.* An assumption here is that there already exists a "gold standard" measure of disordered sleep for each condition. PSG is widely held as the Gold standard for sleep but (a) it is commonly reported subjectively (normal/abnormal); (b) it requires admission to a laboratory and so sleep is in unaccustomed settings with imposed sleep regimen; (c) it has scores for periodic limb movements and arousals but is weighted toward sleep apnoea. A common alternative is to use validated patient reported sleep scales. The Epworth sleepiness score (ESS) is an example for day time sleepiness and the Parkinson's Disease Sleep scale - 2 (PDSS 2) is an example for sleep in PD. The PDSS 2 is a comprehensive questionnaire that asks about night time sleep patterns and day time sleep patterns. It has the short coming that it is self reported, it covers more than night time sleep and it is non linear. This is important because "normal sleep" receives a score of "0" even though normal sleep has a wide range of variability and the transition from normal to moderate is by an increment of "2" and so also is the transition from moderate to severe (ie not linear).

To examine the weightings to apply to variables we have examined the six above-described PKG variables in a) PwP and b) subjects undergoing PSG for a sleep disorder (usually sleep apnoea). We have compared all six variables and, by inspection, chosen to weight those variables that have the greatest variation from controls. We have then iteratively applied different weightings to obtain the greatest correlation with the existing sleep standard (PDSS 2 or PSG).

COMPARISON OF SLEEP DATA FROM NORMAL CONTROLS AND PwP.

Time of arising and retiring. There was a statistically significant likelihood of PwP to go to bed close to 23:00 and arise before 06:00 but the effect size (ie number of minutes difference) was not very meaningful and this was borne about by a non-significant trend p=0.51) for PwP to have a shorter time attempting sleep (Figure 11H). Interestingly, there was non-significant trend to difference in the percent time Active (p=0.52 Mann Whitney) due to much greater variability in patients.

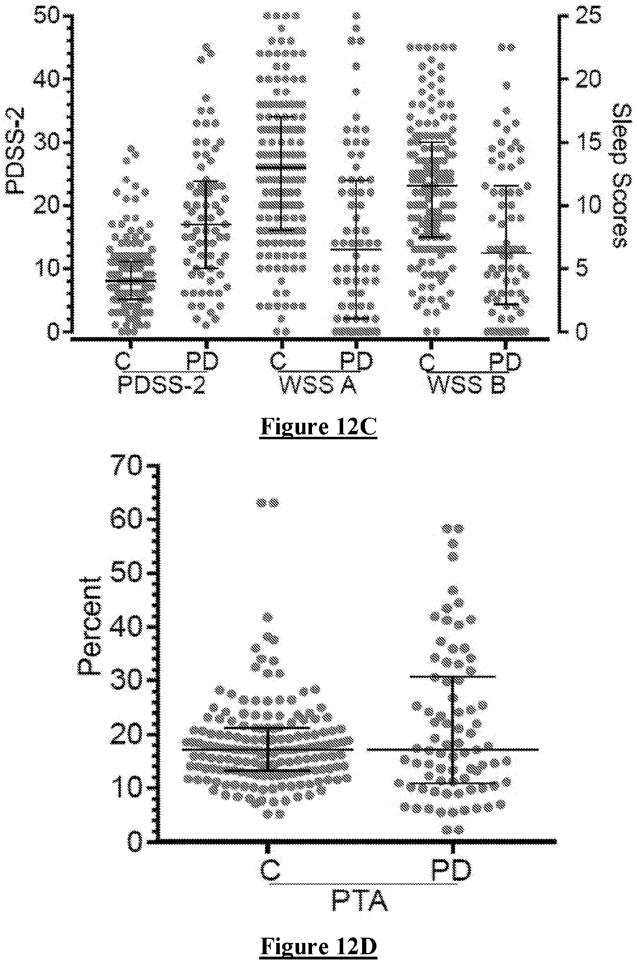

Sleep Efficiency. Differences in the three measures of efficiency (PTI, PTIn and Sleep Duration) are shown in Figure 12A and B and in Table 4. In particular, Figures 12 A, B, & C show the values for Controls (C, green dots) and PwP (PD, red dots) for all of the variables used to create a sleep score (SS, in Fig 12 C, right Y axis). These variables are described in the text and are Sleep Duration (A), Median Fragment Length (MFL, Fig 12B), PTI, PTIn, Sleep Quality (Fig 12B). PDSS-2 was obtained by questionnaire for most PwP and Controls and is shown in Figure 12C. In all graphs there was a significant difference between data from Controls and PwP (>0.0001, Mann Whitney). The differences between Controls and PwP were statistically different and with meaningful effect size. In Summary, PwP spent more time awake but Inactive, less time Immobile and with Shorter Sleep Duration.

Table 4: Scores that contribute to the Sleep Score

| | | | | | PERCENTILE | | | |
|---|---|---|---|---|---|---|---|---|
| | | Min | 10% | 25% | Median | 75% | 90% | Max |
| PTA | C | 5.3 | 10.2 | 13.2 | 17.1 | 21.1 | 26.4 | 63.1 |
| | PD | 2.3 | 7.4 | 9.5 | 12.6 | 17.5 | 24 | 40.6 |
| PTIn | C | 5 | 7 | 10 | 13 | 18 | 24 | 41 |
| | PD | 3.2 | 8 | 13 | 20 | 28 | 40 | 58 |
| PTI | C | 23 | 56 | 61.2 | 69 | 75 | 79 | 88 |
| | PD | 1.5 | 31 | 45 | 59.1 | 75.3 | 80.3 | 90.3 |
| Sleep Quality | C | 31 | 57 | 68 | 77 | 83 | 88 | 93 |
| | PD | 13 | 33 | 45 | 60 | 73 | 81 | 98 |
| Sleep Duration | C | 90 | 217 | 267 | 310 | 350 | 376 | 405 |
| | PD | 2 | 105 | 167 | 257 | 307 | 369 | 405 |
| MFL | C | 8 | 19.3 | 265 | 38 | 60 | 86 | 227 |
| | PD | 8 | 12 | 16 | 25 | 36 | 82 | 368 |
| Immobile 25 | C | 89 | 100 | 106 | 111 | 115 | 119 | 129 |
| | PD | 85 | 89 | 94 | 101 | 109 | 113 | 142 |
| Sleep Score | C | 0 | 5 | 8 | 13 | 17 | 21 | 25 |
| revised PD | PD | 0 | 0 | 1 | 6.5 | 12 | 17 | 25 |

Fragmentation was assessed by the Median Fragment Length (MFL) of each Sleep Fragment. This was significantly shorter in PwP (Figure 12A and B and in Table 4).

Sleep Architecture was measured by Sleep Quality, which measures the proportion of Immobile BKS (>80) that are very Immobile (>110, or higher than $Immobile_{25}$) (Figure 12A and B and in Table 4). This was statistically and meaningfully less in PwP than in Controls.

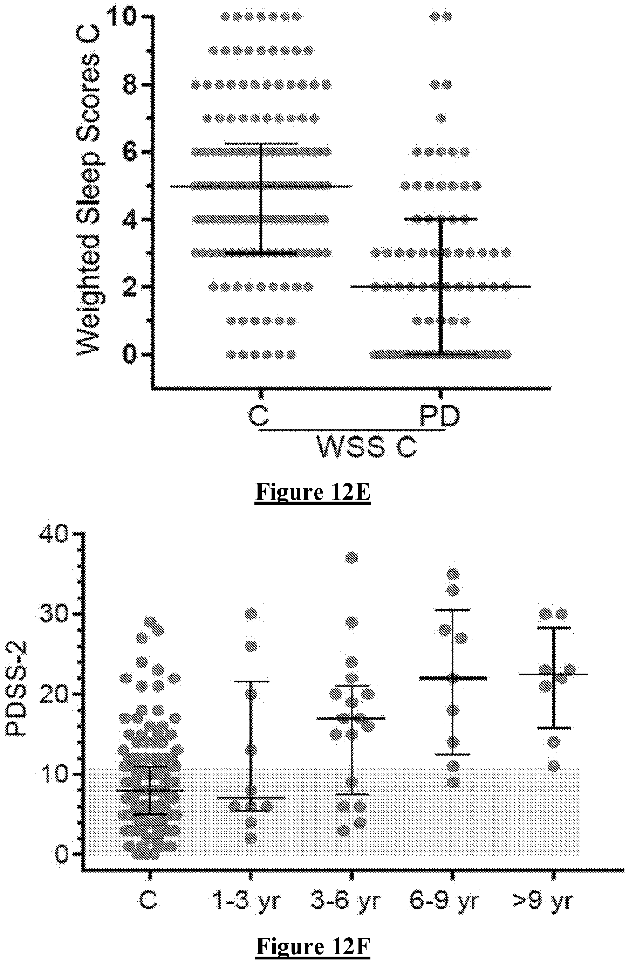

Time Awake was measured using PTA (figure 12 D). In more detail Figure 12 D & E show the PDSS-2 (D) and SS (E) of PwP plotted against duration of disease (years). Controls are shown (C) as a series of bars showing 10th,25th, 50th,75th &90th percentiles. The grey bars show the regions above the 90th percentile (PDSS-2) or below the 10th percentile (SS). There is a trend toward abnormal scores within the first three years of disease. The mean was not statistically different from Controls although the spread was substantially greater in PD subjects as manifest by the larger interquartile range.

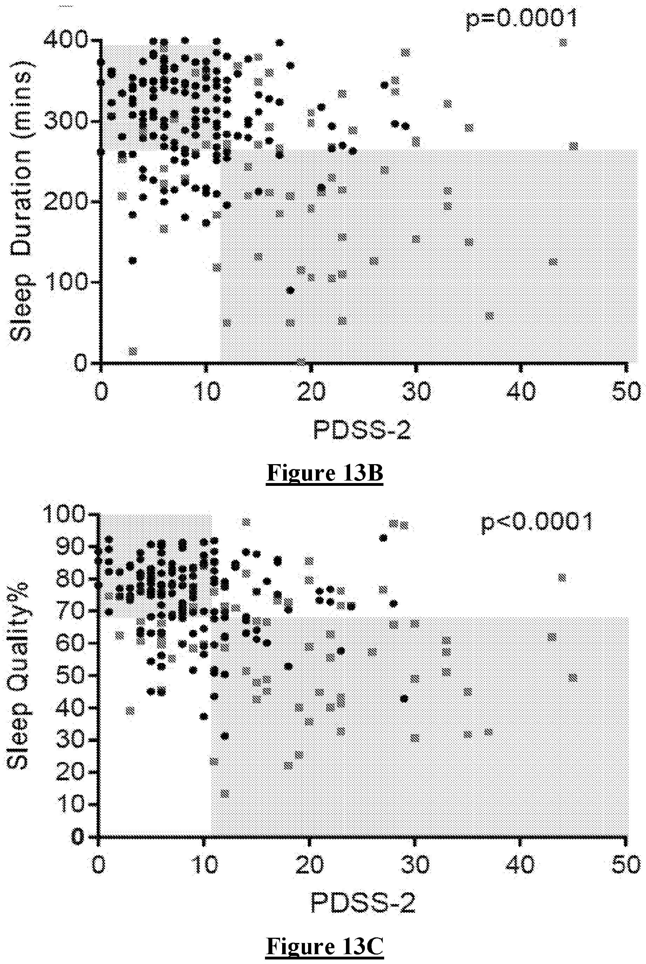

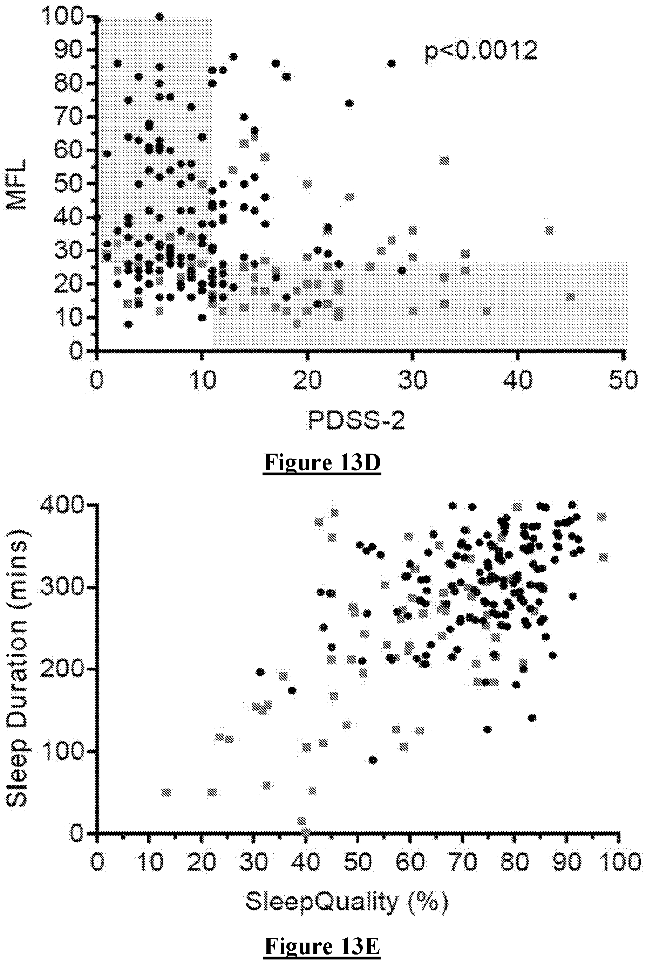

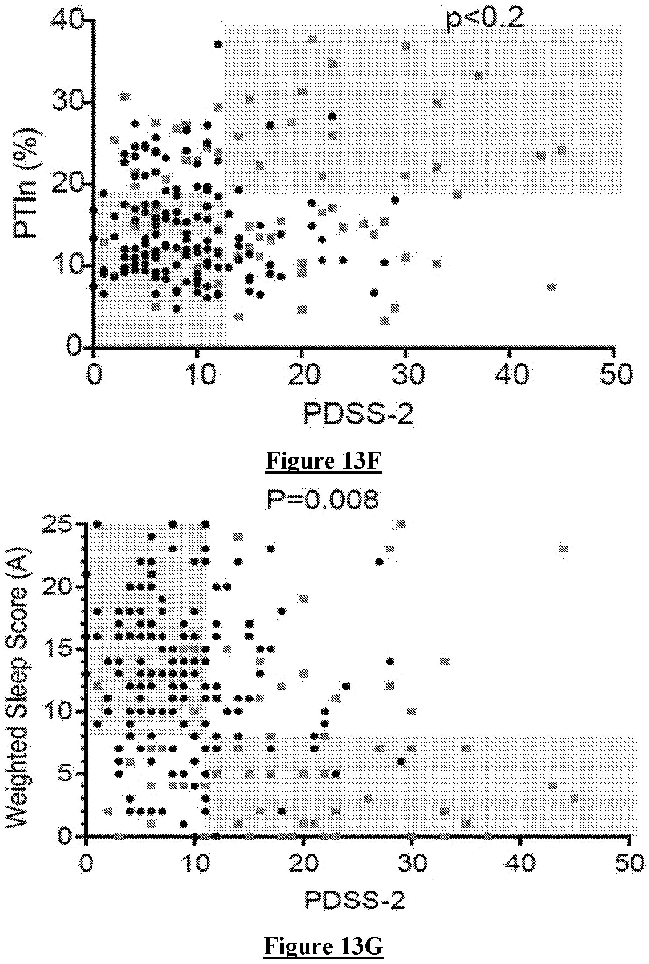

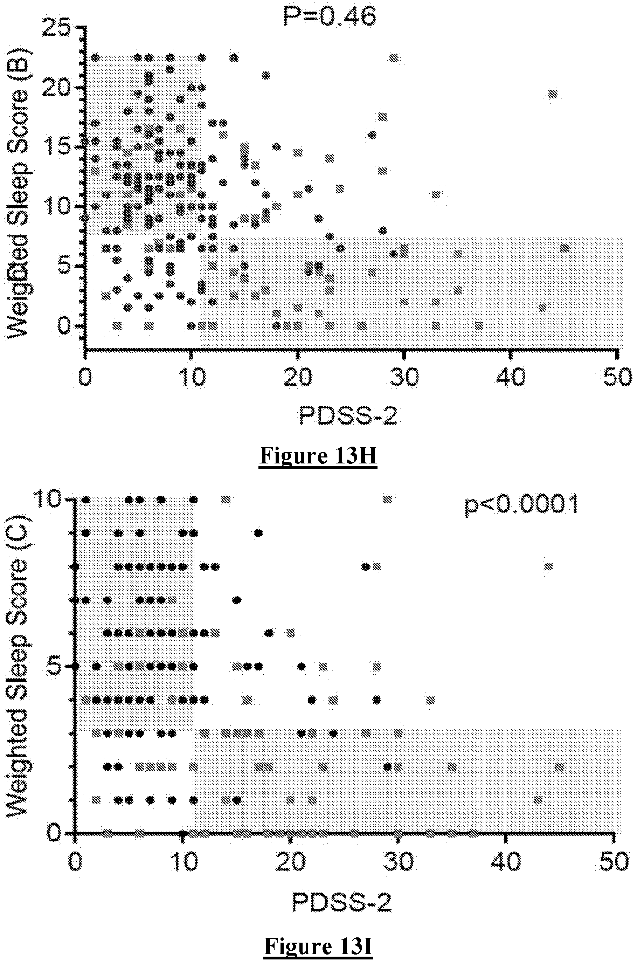

Comparison of each variable with PDSS 2. PDSS 2 is a recognised Sleep Scale. While it is not expected that there will be very high correlation between each variable and the PDSS 2 they should each have a relevant trend if they are likely to influence a Weighted Sleep Score. Each Variable was compared with the PDSS 2 (Figure 13A-F, in which circular data points are controls and square data points are PwP). In more detail Figure 13A shows the relationship between Sleep Score and PDSS-2. The relationship is not significant. Figures 13 B, C, D, F & G show the relationship between PDSS 2 and each subcomponent of the Sleep Score. For Sleep Duration and Sleep Quality individually (B and C) and together (G) there is a significant relationship measured by the Fisher's exact test and with the boundaries set at the 25th percentile of each variable (including the PDSS 2). Note that sleep duration and quality are broadly related (E). There is positive relationship between PDSS 2 and MFL but not with PTIn. In most instances, the relationship was not linear so the correlation was tested using a Fishers exact test with the 75th percentile of each relevant test used as the boundary (grey boxes). This reveals that Sleep Quality, Sleep Duration and MFL best correlated with the PDSS 2 although there was a weaker relationship with the other variables.

Sleep Scores. Using the Sleep Score Formula described above three different Weighted Sleep Scores (WSS) were produced according to the weightings in the table below.

[0094] $WSS = a \times PTA + b \times PTI + c \times, PTIn + d \times \text{Sleep Duration} + e \times MFL + f \times \text{Sleep Quality}$

|  | Weighting | | | | | |
|---|---|---|---|---|---|---|
|  | PTA | PTI | PTIn | Sleep Duration | MFL | Sleep Quality |
| WSS A | 0 | 1 | 1 | 1 | 1 | 1 |
| WSS B | 1 | 0.5 | 0 | 1.5 | 0 | 1.5 |
| WSS C | 0 | 0 | 0 | 1 | 0 | 1 |
| WSS D | 0 | 1 | 0 | 1 | 0 | 1 |
| WSS= Weighted Sleep Score | | | | | | |

WSS C was produced because Sleep Quality and Duration both showed a Good relationship with PDSS 2. WSS B was produced because it was developed for testing against PSG.

The relationship between each WSS and the PDSS 2 is shown in Figure 13G-I. This shows that WSS C produced the best relationship with PDSS 2.

It is notable that MLF also had a good relation with PDSS 2. We predict that using Machine Learning or some other iterative method and a larger data base, better correlation with PDSS 2 and a weighted sleep score will be produced.

The PDSS-2 and WSS were plotted against duration of disease (Figure 12F-I). All Measures showed a similar trend for Sleep states to worsen as diseased progressed. WSS C was most similar to the PDSS 2.

Sleep Scores and Polysomnography. This was a study of 36 subjects who were investigate with a sleep study (mostly for sleep apnoea) and found to have 10 subjects with a normal sleep study (7 as part of a spate research study). Their sleep was grades according to the report from the PSG and scored according to the table below. Note that in Mild - there was some extra comment other "normal" (eg Normal but fragmented sleep) and so they had a separate category but in some cases could have been normal.

| PSG Δ | Controls | Mild - | Mild plus | mild-mod | Mod | Severe minus | Severe |
|---|---|---|---|---|---|---|---|
| Score | 0 | 1 | 2 | 3 | 4 | 5 | 6 |

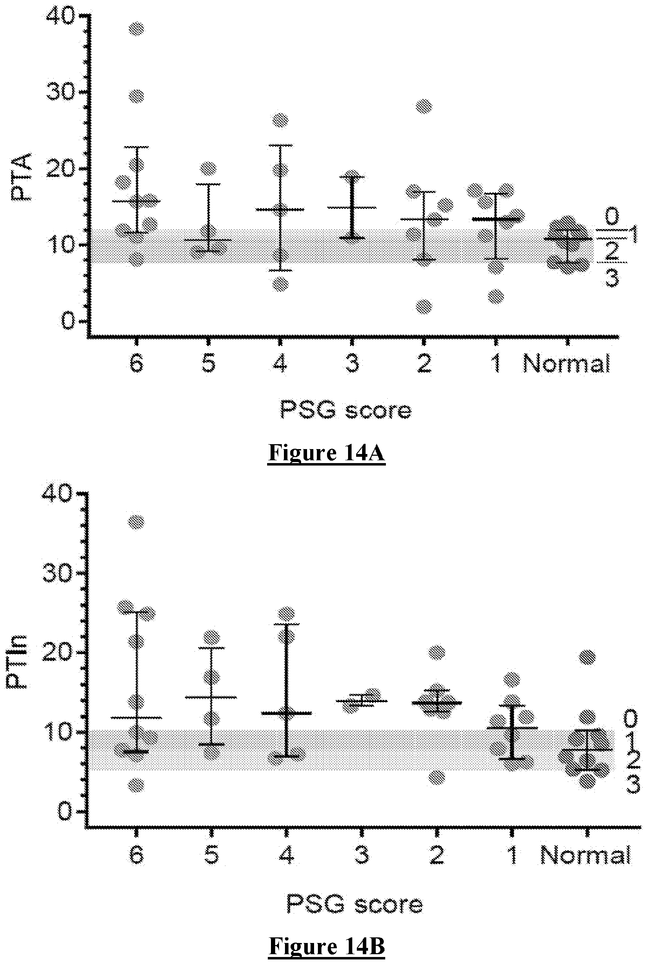

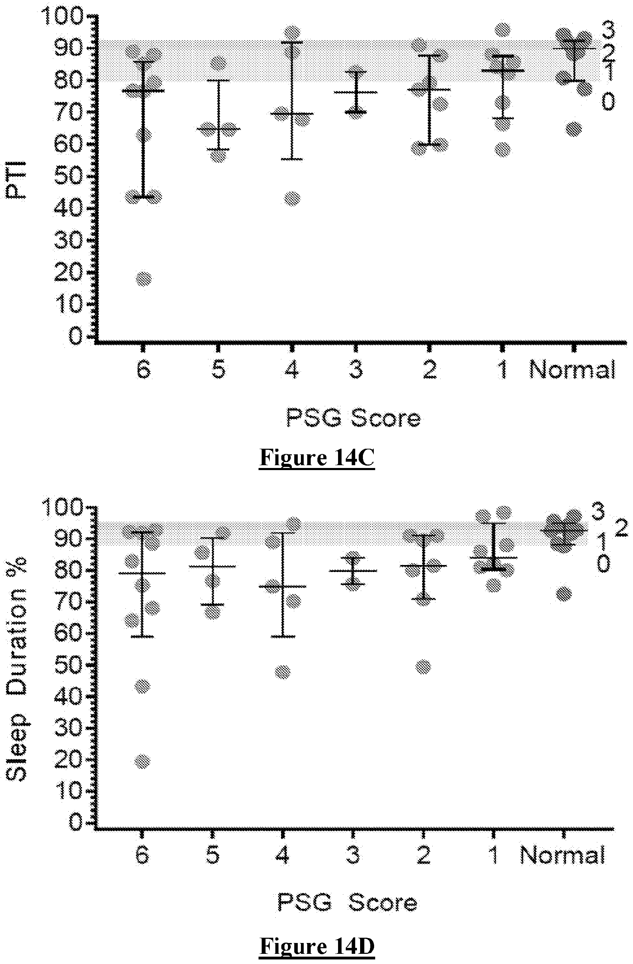

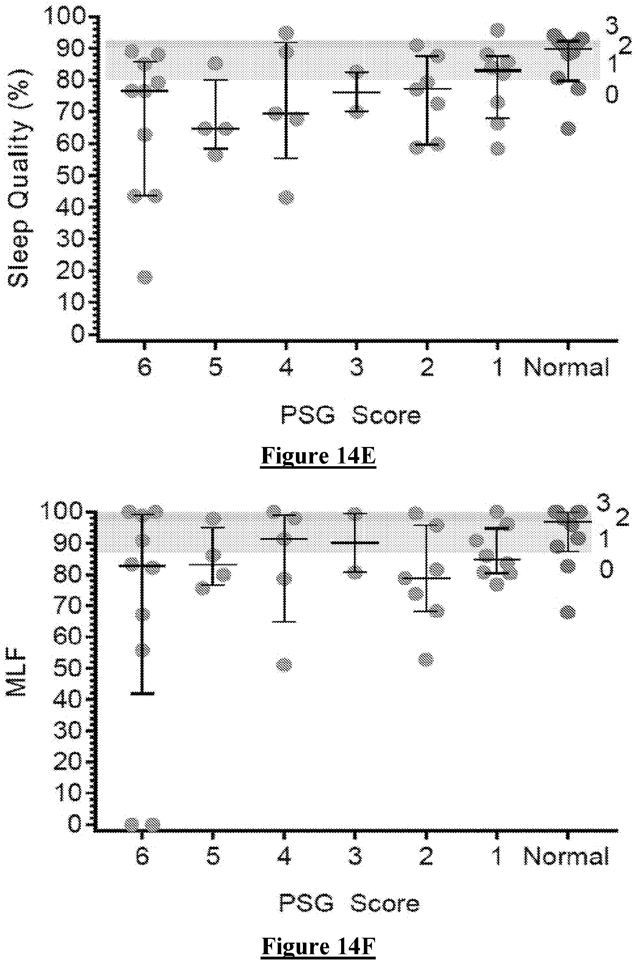

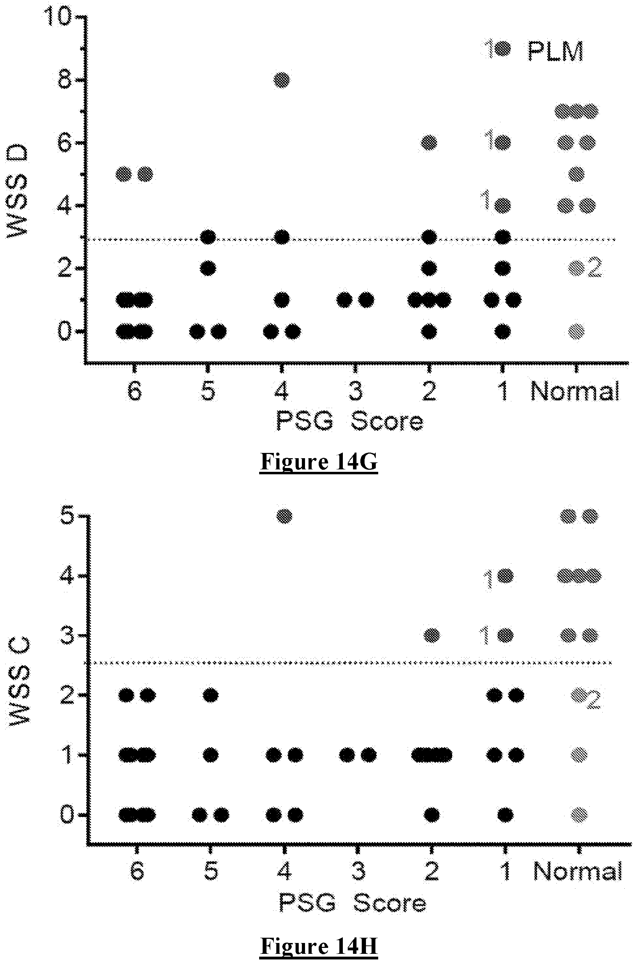

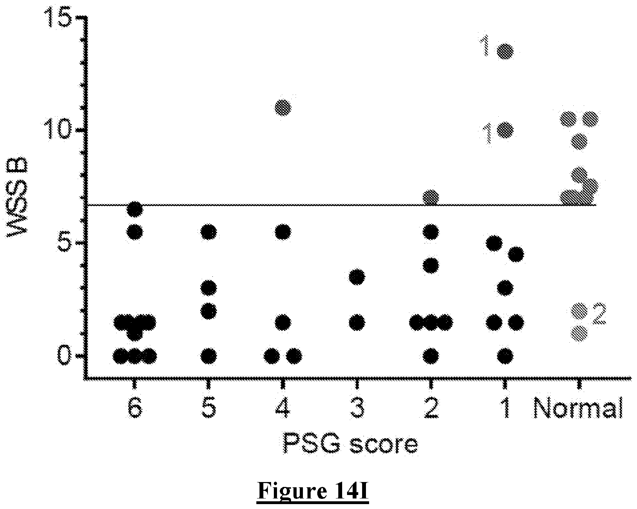

They wore a PKG for the duration of the sleep study and the same sleep variables described above were examined. However, Sleep Duration was now from the time of "lights out" to lights on" in the laboratory and was expressed as Percent time asleep, because this interval varied in time. The relationship between each variable and the PSG score is shown in Figure 14. In each of these graphs the Normal subjects are shown as red dots. The grey band represents the scores between the 25th and 50th percentile of controls and received a score of "1". The orange band represents the scores between the 75th and 50th percentile of controls and received a score of "2". Above the 75th received a score of 3 and below the 25th received a score of "0".

These variables were then summed into a weighted Sleep Score (WSS) to best optimise the capacity to separate Normal subjects from those classed as having anabnormal PSG (Figure 6H-J). In the three figures, black dots represent subjects classified as having abnormal sleep by both the PSG and the PKG scores. Those in Burgundy are false "normal" and in orange false "abnormal" by the PSG relative to the PSG. The subjects marked by a "1" may well be normal (as are indeed all the subjects marked with a PSG score of "1"). The subject marked with a "2" was reported as normal but had a very high Periodic limb movement score on the PSG.

Noting that the best use of the PKG score with reference to the PSG is as a screening tool, it is best to minimise the cases falsely classed as normal and thus WSS B and WSS C provide similar outcomes. These scores were also the best in terms of the PDSS 2 and it is relevant that the 25th percentile cut off used in the PDSS 2 analyses is almost identical to the cut-off in the relevant comparison with the PSG.

Accordingly, it can be concluded that we can predict sleep using a weighting of six variables, and the weights of the variables can vary (in this current form from 0-1.5). The choice of weighting is variable and is currently chosen by inspection of the graphs and iterative application to achieve an optimal relationship. However a machine learning approach is a more sophisticated application of the same approach but allows on going improvement as data becomes available.

These studies further reveal that BKS has ranges (at least four). Immobility induced by sleep is more than just "still" measured by a higher BKS but includes various grades of two or more levels of "stillness" as measured by a higher BKS. Quality of sleep has a relationship to the extent of "stillness" measured by a higher BKS. We believe that this is related to the architecture of sleep.

Fragmentation is a measure of poor sleep. The length of passage of immobility as measured by the number of consecutive BKS that are greater than some specified BKS value (eg 80 or 110) indicates better sleep. The total duration of sleep (using various analyses of BKS to find a total amount of immobility in a specified period of attempted sleep) is a measure of the quality of sleep.

The amount of time with "Active" BKS indicates movements during a night period that suggest either that sleep is not being attempted (poor sleep hygiene) or that movements are intruding into and disrupting sleep (eg REM sleep disorder).

The amount of "Inactive" BKS indicates movements during a night period that suggest either that sleep is being attempted but not achieved (insomnia) or that movements are intruding into and disrupting sleep (eg micro-arousals and periodic limb movements).

These aspects of immobility and Mobility during the night period can be assessed with continuous variables (eg Fragmentation by Median Fragment length), Sleep architecture by measuring the 25th percentile of the BKS value during sleep (ie how still was a person), sleep duration etc). Scores can be given according to the values that represent percentiles (eg 10th,25th,50th,75th,90th) of control subjects to produce a score for each variable. These variables can be weighted, summed and/or combined by any other suitable mathematical function in order to produce a Sleep Score.

There is a difficulty in validating these score because of the problem of a "gold Standard". One gold Standard is the Polysomnogram and another is sleep scales (eg PDSS 2 of PD). Each has their problems. Polysomnogram. Admitted for one night in unfamiliar surroundings and is highly geared toward measuring sleep apnoea and abnormal sleep (ie not normal sleep). Scales of severity are often binary or descriptive and biased toward sleep apnoea. PDSS 2. Is a questionnaire and biased toward PD sleep problems including daytime sleep and pain.

Nevertheless, we can show that in the case of PD, each subcomponent is significantly different in PD subjects from controls (albeit with overlap - but not all PD have sleep problems and not all Controls do not have sleep problem). Furthermore our scale worsens as disease progresses and there is a correlation with some subcomponents with the PDSS 2. This may suggest which problems the PDSS 2 favours (or are more important in PD). In the case of PSG, we can predict with high (but not perfect) accuracy who will be abnormal. Our conclusion is that the existing measures are disease specific and that we can provide sub-scores and total scores that indicate sleep pathology and can be used qualitatively and quantitatively. Using the weightings and the different measures is novel and of value.

While the described embodiments are directed to the identification of normal and abnormal sleep in the context of PD in particular, it is to be appreciated that alternative embodiments of the invention may be applied to identify sleep abnormalities arising from other conditions and in particular non-apnoea sleep abnormalities. For example, it is to be noted that the non-PD control group data, such as found in Figures 7D and 8C, included instances of sleep disturbances which may in other embodiments of the invention be correlated with other conditions by derivation of suitable sleep variable weightings optimised for such conditions in the manner described herein. In particular, non-apnoea conditions may be assessed in this manner.

Reference herein to a "module" may be to a hardware or software structure which is part of a broader structure, and which receives, processes, stores and/or outputs communications or data in an interconnected manner with other system components in order to effect the described functionality.

Some embodiments of the invention may employ kinetic state or sleep state assessment in accordance with any or all of the teaching of International Patent Publication No. WO 2009/149520 by the present applicant.

Thus accelerometry using the Parkinson Kinetigraph (PKG, from Global Kinetics) can be used to distinguish between normal and abnormal sleep in Parkinson's Disease (PD).

It will be appreciated by persons skilled in the art that numerous variations and/or modifications may be made to the invention as shown in the specific embodiments without departing from the scope of the invention as defined by the appended claims. The present embodiments are, therefore, to be considered in all respects as illustrative and not restrictive.

**Claims**

1. A computer-implemented method of assessing sleep state of an individual (112), the method comprising:

   obtaining a time series of accelerometer data from an accelerometer device (15) mounted upon or to the individual (112);
   determining from the time series of accelerometer data a Percent Time Asleep (%TA), wherein the %TA is a percentage of time in which the individual (112) is substantially immobile during a period of attempted sleep;
   **characterized in that** the method further comprises:

   determining from the time series of accelerometer data a Median Time Immobile (MTI), wherein the MTI is a median time of each of the periods in which the individual (112) is substantially immobile during the period of attempted sleep;
   combining the %TA and MTI to produce a sleep score for determining if the attempted sleep by the individual (112) is normal or abnormal based on comparison with a threshold score; and
   if the sleep score exceeds the threshold score, outputting an indication that the attempted sleep by the individual (112) is normal.

2. The method of claim 1 when used in a non-clinical setting such as the individual's home.

3. The method of claim 1 or claim 2 applied to assess sleep state of Parkinsonian subjects.

4. The method of claim 1 or claim 2 applied to assess sleep state of non-Parkinsonian subjects.

5. The method of any one of claims 1 to 4 wherein producing the sleep score further comprises summing or otherwise combining 2 or more of a set of sleep-related variables derived from the accelerometer data.

6. The method of claim 5 wherein the sleep related variables include a variable reflecting the individual's attempts at being active.

7. The method of claim 5 or claim 6 wherein the sleep related variables include a variable reflecting the individual's inactivity while awake.

8. The method of any one of claims 5 to 7 wherein the sleep related variables include a variable reflecting the individual's immobility while asleep.

9. The method of any one of claims 5 to 8 wherein the sleep related variables include a variable reflecting the individual's sleep duration.

10. The method of any one of claims 5 to 9 wherein the sleep related variables include a variable reflecting the individual's sleep fragment length.

11. The method of any one of claims 5 to 10 wherein the sleep related variables include a variable reflecting the individual's Sleep Quality.

**12.** A system for assessing sleep state of an individual (112), the system comprising:

an accelerometer device (15) configured to be mounted upon or to the individual (112) and configured to obtain a time series of accelerometer data; and

a processor (114) configured to:

determine from the time series of accelerometer data a Percent Time Asleep (%TA), wherein the %TA is a percentage of time in which the individual (112) is substantially immobile during a period of attempted sleep;

**characterized in that** the processor (114) is further configured to:

determine from the time series of accelerometer data a Median Time Immobile (MTI), wherein the MTI is a median time of each of the periods in which the individual (112) is substantially immobile during the period of attempted sleep;

combine the %TA and MTI to produce a sleep score for determining if the attempted sleep by the individual (112) is normal or abnormal based on comparison with a threshold score; and

if the sleep score exceeds the threshold score, output an indication that the attempted sleep by the individual (112) is normal.

**13.** A non-transitory computer readable medium for assessing sleep state of an individual (112), comprising instructions which, when executed by one or more processors (114), cause said one or more processors to perform the following steps:

obtaining a time series of accelerometer data from an accelerometer device (15) mounted upon or to the individual (112);

determining from the time series of accelerometer data a Percent Time Asleep (%TA), wherein the %TA is a percentage of time in which the individual (112) is substantially immobile during a period of attempted sleep;

**characterized in that** the instructions, when executed by the one or more processors (114), further cause said one or more processors to perform the following:

determining from the time series of accelerometer data a Median Time Immobile (MTI), wherein the MTI is a median time of each of the periods in which the individual (112) is substantially immobile during the period of attempted sleep;

combining the %TA and MTI to produce a sleep score for determining if the attempted sleep by the individual is normal or abnormal based on comparison with a threshold score; and

if the sleep score exceeds the threshold score, outputting an indication that the attempted sleep by the individual (112) is normal.

**Patentansprüche**

**1.** Ein computerimplementiertes Verfahren zum Überprüfen eines Schlafzustands von einem Individuum (112), wobei das Verfahren aufweist:

Erhalten einer Zeitfolge von Beschleunigungsdaten von einer Beschleunigungsvorrichtung (15), die auf oder an dem Individuum (112) angebracht ist;

Bestimmen aus der Zeitfolge von Beschleunigungsdaten einer prozentualen Einschlafzeit (%TA), wobei die %TA ein Prozentsatz einer Zeit ist, in der das Individuum (112) während einer Zeitdauer eines Schlafversuchs im Wesentlichen unbeweglich ist;

**dadurch gekennzeichnet, dass** das Verfahren ferner aufweist:

Bestimmen aus der Zeitfolge von Beschleunigungsdaten einer mittleren Unbeweglichkeitszeit (MTI), wobei die MTI eine mittlere Zeit von jeder der Zeitdauern ist, in der das Individuum (112) während der Zeitdauer eines Schlafversuchs im Wesentlichen unbeweglich ist;

Kombinieren der %TA und MTI, um einen Schlafwert zu erzeugen, um basierend auf einem Vergleich mit einem Schwellenwert zu bestimmen, falls der Schlafversuch durch das Individuum (112) normal oder anormal ist; und

falls der Schlafwert den Schwellenwert überschreitet, Ausgeben eines Hinweises, dass der Schlafversuch durch das Individuum (112) normal ist.

2. Das Verfahren nach Anspruch 1 bei Verwendung in einem nichtklinischen Umfeld, wie etwa das Haus bzw. die Wohnung des Individuums.

3. Das Verfahren nach Anspruch 1 oder 2 bei Anwendung zur Überprüfung eines Schlafzustands von Parkinsonsubjekten.

4. Das Verfahren nach Anspruch 1 oder 2 bei Anwendung zur Überprüfung eines Schlafzustands von Nicht-Parkinsonsubjekten.

5. Das Verfahren nach einem der Ansprüche 1-4, wobei das Erzeugen des Schlafwerts ferner ein Summieren oder andernfalls ein Kombinieren von 2 oder mehr aus einem Satz an schlafbezogenen Variablen aufweist, die aus den Beschleunigungsdaten abgeleitet sind.

6. Das Verfahren nach Anspruch 5, wobei die schlafbezogenen Variablen eine Variable umfassen, die Aktivitätsversuche des Individuums darstellt.

7. Das Verfahren nach Anspruch 5 oder 6, wobei die schlafbezogenen Variablen eine Variable umfassen, die eine Inaktivität des Individuums darstellt, während es wach ist.

8. Das Verfahren nach einem der Ansprüche 5-7, wobei die schlafbezogenen Variablen eine Variable umfassen, die eine Unbeweglichkeit des Individuums darstellt, während es schläft.

9. Das Verfahren nach einem der Ansprüche 5-8, wobei die schlafbezogenen Variablen eine Variable umfassen, die eine Schlafdauer des Individuums darstellt.

10. Das Verfahren nach einem der Ansprüche 5-9, wobei die schlafbezogenen Variablen eine Variable umfassen, die eine Schlafunterbrechungsdauer des Individuums darstellt.

11. Das Verfahren nach einem der Ansprüche 5-10, wobei die schlafbezogenen Variablen eine Variable umfassen, die eine Schlafqualität des Individuums darstellt.

12. Ein System zum Überprüfen eines Schlafzustands von einem Individuum (112), wobei das System aufweist:

   eine Beschleunigungsvorrichtung (15), die konfiguriert ist, um auf oder an dem Individuum (112) angebracht zu sein, und die konfiguriert ist, um eine Zeitfolge von Beschleunigungsdaten zu erhalten; und
   einen Prozessor (114), der konfiguriert ist, um:
   aus der Zeitfolge von Beschleunigungsdaten eine prozentuale Einschlafzeit (%TA) zu bestimmen, wobei die %TA ein Prozentsatz einer Zeit ist, in der das Individuum (112) während einer Zeitdauer eines Schlafversuchs im Wesentlichen unbeweglich ist;
   **dadurch gekennzeichnet, dass** der Prozessor (114) ferner konfiguriert ist, um:

      aus der Zeitfolge von Beschleunigungsdaten eine mittlere Unbeweglichkeitszeit (MTI) zu bestimmen, wobei die MTI eine mittlere Zeit von jeder der Zeitdauern ist, in der das Individuum (112) während der Zeitdauer eines Schlafversuchs im Wesentlichen unbeweglich ist;
      die %TA und MTI zu kombinieren, um einen Schlafwert zu erzeugen, um basierend auf einem Vergleich mit einem Schwellenwert zu bestimmen, falls der Schlafversuch durch das Individuum (112) normal oder anormal ist; und
      falls der Schlafwert den Schwellenwert überschreitet, einen Hinweis auszugeben, dass der Schlafversuch durch das Individuum (112) normal ist.

13. Ein nicht-transitorisches computerlesbares Medium zum Überprüfen eines Schlafzustands von einem Individuum (112), mit Instruktionen, die bei Ausführung durch einen oder mehrere Prozessoren (114) die Prozessoren veranlassen, die folgenden Schritte durchzuführen:

   Erhalten einer Zeitfolge von Beschleunigungsdaten von einer Beschleunigungsvorrichtung (15), die auf oder an dem Individuum (112) angebracht ist;
   Bestimmen aus der Zeitfolge von Beschleunigungsdaten einer prozentualen Einschlafzeit (%TA), wobei die %TA ein Prozentsatz einer Zeit ist, in der das Individuum (112) während einer Zeitdauer eines Schlafversuchs im Wesentlichen unbeweglich ist;

**dadurch gekennzeichnet, dass** die Instruktionen bei Ausführung durch den einen oder die mehreren Prozessoren (114) die Prozessoren ferner veranlassen, die folgenden Schritte durchzuführen:

Bestimmen aus der Zeitfolge von Beschleunigungsdaten einer mittleren Unbeweglichkeitszeit (MTI), wobei die MTI eine mittlere Zeit von jeder der Zeitdauern ist, in der das Individuum (112) während der Zeitdauer eines Schlafversuchs im Wesentlichen unbeweglich ist;

Kombinieren der %TA und MTI, um einen Schlafwert zu erzeugen, um basierend auf einem Vergleich mit einem Schwellenwert zu bestimmen, falls der Schlafversuch durch das Individuum normal oder anormal ist; und

falls der Schlafwert den Schwellenwert überschreitet, Ausgeben eines Hinweises, dass der Schlafversuch durch das Individuum (112) normal ist.

**Revendications**

1. Procédé mis en œuvre par ordinateur d'évaluation de l'état de sommeil d'un individu (112), le procédé comprenant :

l'obtention d'une série temporelle de données d'accéléromètre à partir d'un dispositif d'accéléromètre (15) monté sur ou vers l'individu (112) ;

la détermination à partir de la série temporelle de données d'accéléromètre d'un pourcentage de temps de sommeil (%TA), dans lequel le %TA est un pourcentage de temps au cours duquel l'individu (112) est sensiblement immobile pendant une période de tentative de sommeil ;

**caractérisé en ce que** le procédé comprend en outre :

la détermination à partir de la série temporelle de données d'accéléromètre d'un temps médian d'immobilité (MTI), dans lequel le MTI est un temps médian de chacune des périodes au cours desquelles l'individu (112) est sensiblement immobile pendant la période de tentative de sommeil ;

la combinaison des %TA et MTI pour produire un score de sommeil pour déterminer si la tentative de sommeil par l'individu (112) est normale ou anormale sur la base de la comparaison avec un score seuil ; et si le score de sommeil dépasse le score seuil, l'émission d'une indication que la tentative de sommeil par l'individu (112) est normale.

2. Procédé selon la revendication 1 lorsqu'il est utilisé dans un cadre non clinique tel que le logement d'un individu.

3. Procédé selon la revendication 1 ou la revendication 2 appliqué pour évaluer l'état de sommeil de sujets parkinsoniens.

4. Procédé selon la revendication 1 ou la revendication 2 appliqué pour évaluer l'état de sommeil de sujets non parkinsoniens.

5. Procédé selon l'une quelconque des revendications 1 à 4, dans lequel la production du score de sommeil comprend en outre l'addition ou sinon la combinaison de 2 ou plus d'un ensemble de variables liées au sommeil dérivées des données d'accéléromètre.

6. Procédé selon la revendication 5, dans lequel les variables liées au sommeil comportent une variable reflétant les tentatives de l'individu à être actif.

7. Procédé selon la revendication 5 ou la revendication 6, dans lequel les variables liées au sommeil comportent une variable reflétant l'inactivité de l'individu pendant qu'il est éveillé.

8. Procédé selon l'une quelconque des revendications 5 à 7, dans lequel les variables liées au sommeil comportent une variable reflétant l'immobilité de l'individu pendant qu'il est endormi.

9. Procédé selon l'une quelconque des revendications 5 à 8, dans lequel les variables liées au sommeil comportent une variable reflétant la durée du sommeil de l'individu.

10. Procédé selon l'une quelconque des revendications 5 à 9, dans lequel les variables liées au sommeil comportent une variable reflétant la longueur de fragment de sommeil de l'individu.

11. Procédé selon l'une quelconque des revendications 5 à 10, dans lequel les variables liées au sommeil comportent une variable reflétant la qualité du sommeil de l'individu.

12. Système pour évaluer l'état de sommeil d'un individu (112), le système comprenant :

un dispositif d'accéléromètre (15) configuré pour être monté sur ou vers l'individu (112) et configuré pour obtenir une série temporelle de données d'accéléromètre ; et
un processeur (114) configuré pour :
déterminer à partir de la série temporelle de données d'accéléromètre un pourcentage de temps de sommeil (%TA), dans lequel le %TA est un pourcentage de temps au cours duquel l'individu (112) est sensiblement immobile pendant une période de tentative de sommeil ;
**caractérisé en ce que** le processeur (114) est en outre configuré pour :

déterminer à partir de la série temporelle de données d'accéléromètre un temps médian d'immobilité (MTI), dans lequel le MTI est un temps médian de chacune des périodes au cours desquelles l'individu (112) est sensiblement immobile pendant la période de tentative de sommeil ;
combiner les %TA et MTI pour produire un score de sommeil pour déterminer si la tentative de sommeil par l'individu (112) est normale ou anormale sur la base de la comparaison avec un score seuil ; et
si le score de sommeil dépasse le score seuil, émettre une indication que la tentative de sommeil par l'individu (112) est normale.

13. Support lisible par ordinateur non transitoire pour évaluer l'état de sommeil d'un individu (112), comprenant des instructions qui, lorsqu'elles sont exécutées par un ou plusieurs processeurs (114), amènent lesdits un ou plusieurs processeurs à effectuer les étapes suivantes :

l'obtention d'une série temporelle de données d'accéléromètre à partir d'un dispositif d'accéléromètre (15) monté sur ou vers l'individu (112) ;
la détermination à partir de la série temporelle de données d'accéléromètre d'un pourcentage de temps de sommeil (%TA), dans lequel le %TA est un pourcentage de temps au cours duquel l'individu (112) est sensiblement immobile pendant une période de tentative de sommeil ;
**caractérisé en ce que** les instructions, lorsqu'elles sont exécutées par les un ou plusieurs processeurs (114), amènent lesdits un ou plusieurs processeurs à effectuer ce qui suit :

la détermination à partir de la série temporelle de données d'accéléromètre d'un temps médian d'immobilité (MTI), dans lequel le MTI est un temps médian de chacune des périodes au cours desquelles l'individu (112) est sensiblement immobile pendant la période de tentative de sommeil ;
la combinaison des %TA et MTI pour produire un score de sommeil pour déterminer si la tentative de sommeil par l'individu est normale ou anormale sur la base de la comparaison avec un score seuil ; et
si le score de sommeil dépasse le score seuil, l'émission d'une indication que la tentative de sommeil par l'individu (112) est normale.

**Figure 1**

**Figure 2**

**Figure 3**

**Figure 4**

**Figure 5**

**Figure 6**

**Figure 7A**

**Figure 7B**

**Figure 7C**

**Figure 7D**

**Figure 7E**

**Figure 8A**

**Figure 8B**

**Figure 8C**

**Figure 9A**

**Figure 9B**

**Figure 9C**

**Figure 10A**

**Figure 10B**

**Figure 10C**

**Figure 11A**

**Figure 11B**

**Figure 11C**

**Figure 11D**

Figure 11E

Figure 11F

**Figure 11G**

**Figure 11H**

**Figure 12A**

**Figure 12B**

**Figure 12C**

**Figure 12D**

**Figure 12E**

**Figure 12F**

**Figure 12G**

**Figure 12H**

**Figure 12I**

p=0.02

PDSS-2

**Figure 13A**

**Figure 13B**

**Figure 13C**

**Figure 13D**

**Figure 13E**

**Figure 13F**

**Figure 13G**

**Figure 13H**

**Figure 13I**

**Figure 14A**

**Figure 14B**

**Figure 14C**

**Figure 14D**

**Figure 14E**

**Figure 14F**

**Figure 14G**

**Figure 14H**

**Figure 14I**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- AU 2016900036

- WO 2009149520 A

**Non-patent literature cited in the description**

- **JENNIFER GIRSCHIK et al.** Validation of Self-Reported Sleep Against Actigraphy. *Journal of Epidemiology,* 28 July 2012, vol. 22 (5), 462-468

- Sleep in Parkinson's disease: A comparison of actigraphy and subjective measures. **STAVITSKY et al.** Parkinsonism and related disorders. ELSEVIER SCIENCE, 01 May 2010, vol. 16, 280-283